# EUROPEAN PATENT SPECIFICATION

(11) **EP 4 454 212 B1**
(45) Date of publication and mention of the grant of the patent: **08.04.2026**
(21) Application number: 22836232.3
(22) Date of filing: 19.12.2022
(51) Int. Cl.: H04L 9/40, G06Q 10/0833, G06Q 10/08, G06Q 10/00, G06Q 10/06, G06Q 10/087, G06Q 10/30, G06Q 30/018, G06Q 50/04, H04L 9/00

(54) **BATTERY PASSPORT**
BATTERIEPASS
PASSEPORT BATTERIE

(30) Priority: 21.12.2021 EP 21216268; 21.12.2021 EP 21216269; 21.12.2021 EP 21216270; 21.12.2021 EP 21216271; 21.12.2021 EP 21216286; 21.12.2021 EP 21216292; 21.12.2021 EP 21216326; 21.12.2021 EP 21216327; 21.12.2021 EP 21216333; 04.04.2022 EP 22166573; 12.04.2022 EP 22167945; 10.05.2022 EP 22172609; 10.05.2022 EP 22172611; 10.05.2022 EP 22172615; 10.05.2022 EP 22172617; 10.05.2022 EP 22172619; 09.09.2022 EP 22194793; 09.09.2022 EP 22194800; 09.09.2022 EP 22194808; 09.09.2022 EP 22194815; 09.09.2022 EP 22194818; 14.10.2022 EP 22201672; 14.10.2022 US 202263416091 P; 18.10.2022 EP 22202183; 05.12.2022 EP 22211421
(43) Date of publication of application: 30.10.2024
(60) Divisional application: 26161882.1
(73) Proprietor: BASF SE, 67056 Ludwigshafen am Rhein (DE)
(72) Inventor: SCHWABE, Henning, 67056 Ludwigshafen (DE); HAARDT, Dennis, 67056 Ludwigshafen (DE)
(74) Representative: BASF IP Association
(86) International application number: PCT/EP2022/086731
(87) International publication number: WO 2023/117957

(56) References cited:
- CN-A- 109 165 530
- KR-A- 20210 057 233
- NEVINE TEWFIK MCIT-EGYPT: "New Baseline document for draft Recommendation ITU-T L.SM_B "Sustainable Management of Batteries" - Input of Q7/5 e-meeting to be held on 15 July 2021;ID01", vol. 7/5, 15 July 2021 (2021-07-15), pages 1 - 48, XP044313540, Retrieved from the Internet <URL:https://www-api.itu.int/ifa/t/2017/sg5/exchange/wp2/q7/2021/2021-07-15_e-meeting/ID01-ITU-T-L.SM_Batteries_input_15%20July%202021.docx> [retrieved on 20210715]

## Description

### TECHNICAL FIELD

The present disclosure relates to an apparatus for generating a battery passport, a computer-implemented method for generating a battery passport, a respective computer program element, a use of the battery passport, a component of a battery and/or a battery associated with such a battery passport, a battery passport including one or more decentral identifiers and data related to the battery data and methods for producing a battery component associated with such a battery passport.

### TECHNICAL BACKGROUND

In the supply of batteries multiple regulatory requirements need to be met, which differ depending on the chemical product used in the battery. For instance, in automotive supply chains chemical companies provide standardized information using the International Chemical product Data System (IMDS). Such system allows to collect data along the automotive supply chain. Participants in the automotive supply chain register with the IMDS service and maintain product entries in the central database as provided and hosted by a third-party provider.

Systems like IMDS are static regarding data, prone to error and cumbersome in handling or maintenance. Owing to the highly specific and centralized setup of such systems, exchange and sharing of data is laborious. For realizing more efficient and reliable battery production, there is a need to simplify data exchange and sharing between value chain players. CN 109 165 530 A discloses a blockchain is used to track shipment of batteries. Upon the first shipment of a battery, power battery information, such as battery serial number, battery material, cell material etc., is packaged into a header block and broadcast to blockchain nodes. All nodes verify the block and reach a consensus to add it to the blockchain. The battery info can be queried from the blockchain. When the battery is traded, a new block is generated and linked to the previous block on the blockchain.

### SUMMARY OF THE INVENTION

The present invention is defined in the independent claims.

Preferred embodiments are defined in the dependent claims.

In one aspect of the present disclosure, an apparatus for generating a battery passport is provided, the apparatus comprising:
one or more computing nodes and one or more computer-readable media having thereon computer-executable instructions that are structured such that, when executed by the one or more computing nodes, cause the apparatus to perform the following steps:
receiving a request to provide a decentral identifier associated with battery data of at least one component of a battery or of at least a component of a battery;
in response to the request, generating the battery passport including the decentral identifier and data related to the battery data of the at least one component of the battery.

In a further aspect of the present disclosure, a computer-implemented method for generating a battery passport is disclosed, the method comprising:
receiving a request to provide a decentral identifier associated with battery data of at least one component of a battery or of at least a component of a battery;
in response to the request, generating the battery passport including the decentral identifier and data related to the battery data of the at least one component of the battery.

In a further aspect of the present disclosure, an apparatus for generating a battery passport is disclosed, the apparatus comprising:
at least one receiving unit configured to receive a request to provide a decentral identifier associated with battery data of at least one component of a battery or of at least a component of a battery;
at least one generating unit configured to generate the battery passport, in response to the request, including the decentral identifier and data related to the battery data of the at least one component of the battery.

A further aspect of the present disclosure relates to a method for producing or providing a component of a battery associated with a battery passport, the method comprising:
producing the component of the battery connected to or comprising a physical identifier;
assigning the physical identifier to a decentral identifier for generating the battery passport associated with the produced component of the battery,
generating the battery passport according to the computer-implemented method for generating a battery passport or by the system or apparatus for generating a battery passport.

A further aspect of the present disclosure relates to a system for producing or providing a component of a battery associated with a battery passport, the system comprising:
a production line configured to produce the component of the battery connected to or comprising a physical identifier;
an assignor configured to assign the physical identifier to a decentral identifier for generating the battery passport associated with the produced component of the battery;
a battery passport generator configured to generate the battery passport by receiving a request to provide a decentral identifier associated with battery data of at least a component of a battery and in response to the request, generating the battery passport including the decentral identifier and data related to the battery data of the at least one component of the battery.

A further aspect of the present disclosure relates to a computer-Implemented method for producing or providing a component of a battery associated with a battery passport, the method comprising:
based on a physical identifier of the component of the battery, requesting to provide a decentral identifier and assigning the decentral identifier to the physical identifier;
in response to the request, the battery passport including the decentral identifier and data related to the produced component of the battery, preferably the electrode active material, is generated according to the computer-implemented method for generating a battery passport or by the system or apparatus for generating a battery passport.

A further aspect of the present disclosure relates to an apparatus for producing or providing a component of a battery associated with a battery passport, the apparatus comprising:
one or more computing nodes and one or more computer-readable media having thereon computer-executable instructions that are structured such that, when executed by the one or more computing nodes, cause the apparatus to perform the following steps:
based on a physical identifier of the component of the battery, requesting to provide a decentral identifier and assigning the decentral identifier to the physical identifier;
in response to the request, generating the battery passport including the decentral identifier and data related to the produced component of the battery, preferably the electrode active material, wherein the battery passport is generated by receiving a request to provide a decentral identifier associated with battery data of at least the component of the battery and in response to the request, generating the battery passport including the decentral identifier and data related to the battery data of the component of the battery.

A further aspect of the present disclosure relates to an apparatus for producing or providing a component of a battery associated with a battery passport, the apparatus compeiring:
a requestor configured to request, based on a physical identifier of the component of the battery, to provide a decentral identifier;
an assignor configured to assign the decentral identifier to the physical identifier;
a battery passport generator configured to generate, in response to the request, the battery passport including the decentral identifier and data related to the produced component of the battery, preferably the electrode active material, wherein the battery passport is generated by receiving a request to provide a decentral identifier associated with battery data of at least the component of the battery and in response to the request, generating the battery passport including the decentral identifier and data related to the battery data of at least the component of the battery.

In a further aspect an apparatus for producing or providing a component of a battery associated with a battery passport is disclosed, the apparatus comprising:
a collector configured to collect battery data associated with the component of the battery or battery data associated with the production of the component of the battery, wherein the component of the battery is connected to or comprises the physical identifier;
an assignor configured to assign the physical identifier to a decentral identifier for generating the battery passport associated with the produced component of the battery;
a battery passport generator configured to generate the battery passport by receiving a request to provide a decentral identifier associated with battery data of at least the component of the battery and in response to the request, generating the battery passport including the decentral identifier and data related to battery data of at least the component of the battery.

In a further aspect a computer-implemented method for producing or providing a component of a battery associated with a battery passport is disclosed, the method comprising:
collecting battery data associated with the component of the battery or battery data associated with the production of the component of the battery, wherein the component of the battery is connected to or comprises the physical identifier;
assigning the physical identifier to a decentral identifier for generating the battery passport associated with the produced component of the battery;
generating the battery passport includes receiving a request to provide a decentral identifier associated with battery data of at least the component of the battery and in response to the request, generating the battery passport including the decentral identifier and data related to battery data of at least the component of the battery.

In a further aspect a system for producing or providing a component of a battery associated with a battery passport is disclosed, the system comprising:
a production line configured to produce the component of the battery connected to or comprising a physical identifier;
a collector configured to collect battery data associated with the component of the battery or battery data associated with the production of the component of the battery, wherein the component of the battery is connected to or comprises the physical identifier;
an assignor configured to assign the physical identifier to a decentral identifier for generating the battery passport associated with the produced component of the battery;
a battery passport generator configured to generate the battery passport by receiving a request to provide a decentral identifier associated with battery data of at least the component of the battery and in response to the request, generating the battery passport including the decentral identifier and data related to battery data of at least the component of the battery.

In a further aspect a method for producing or providing a component of a battery associated with a battery passport is disclosed, the method comprising:
producing the component of the battery connected to or comprising a physical identifier;
collecting battery data associated with the component of the battery or battery data associated with the production of the component of the battery, wherein the component of the battery is connected to or comprises the physical identifier;
assigning the physical identifier to the decentral identifier for generating the battery passport associated with the produced component of the battery. wherein generating the battery passport includes receiving a request to provide a decentral identifier associated with battery data of at least the component of the battery and in response to the request, generating the battery passport including the decentral identifier and data related to battery data of at least the component of the battery.

A further aspect of the present disclosure relates to a use of the battery passport as generated according to the computer-implemented method for generating a battery passport or by the system or apparatus for generating a battery passport to further process the at least one component of the battery and/or the battery associated with the battery passport.

A further aspect of the present disclosure relates to a use of the battery passport as generated according to the computer-implemented method for producing or providing a battery component associated with a battery passport or by the system or apparatus for producing or providing a battery component associated with a battery passport to further process the at least one component of the battery and/or the battery associated with the battery passport.

A further aspect of the present disclosure relates to a component of a battery and/or a battery associated with the battery passport, wherein the battery passport including one or more decentral identifiers and data related to the battery data is generated according to the computer-implemented method for generating a battery passport or by the system or apparatus for generating a battery passport.

A further aspect of the present disclosure relates to a component of a battery and/or a battery associated with the battery passport, wherein the battery passport including one or more decentral identifiers and data related to the battery data is generated according to the computer-implemented method for producing or providing a battery component associated with a battery passport or by the system or apparatus for producing or providing a battery component associated with a battery passport.

A further aspect of the present disclosure relates to a battery passport including one or more decentral identifier(s) and data related to the battery data, wherein the battery passport is generated according to the computer-implemented method for generating a battery passport or by the system or apparatus for generating a battery passport.

A further aspect of the present disclosure relates to a battery passport including one or more decentral identifier(s) and data related to the battery data, wherein the battery passport is generated according to the computer-implemented method for producing or providing a battery component associated with a battery passport or by the system or apparatus for producing or providing a battery component associated with a battery passport.

A further aspect of the present disclosure relates to a computer program element with instructions, which when executed on one or more computing node(s) is configured to carry out the steps of the computer-implemented method for generating a battery passport or for producing or providing a battery component associated with a battery passport.

Any disclosure and embodiments described herein relate to the methods, the systems, and the computer program elements lined out above and vice versa. Advantageously, the benefits provided by any of the embodiments and examples equally apply to all other embodiments and examples and vice versa.

As used herein "determining" also includes "initiating or causing to determine", "querying" also includes "initiating or causing to query, correlating and/or matching" and "providing" also includes "initiating or causing to determine, generate, select, send or receive". "Initiating or causing to perform an action" includes any processing signal that triggers a computing device to perform the respective action.

The methods, apparatuses, systems and computer elements disclosed herein provide an efficient, secure and robust way for sharing or exchanging data across different participant nodes in battery value chains. In particular, attaching a decentral identifier and associated battery data allows for simplified and customizable data sharing or exchange from battery industry to battery supply chain participants. Therefore, a simpler way for data exchange and sharing between value chain players is provided allowing a more efficient and reliable battery production. A more reliable and efficient further processing of supplied batteries by upstream participants of the battery supply chain can be achieved, while the data may remain in the ownership of the battery supplier supplying the upstream participant. By combining the data related to battery directly with the decentral identifier and optionally one or more authentication mechanisms more reliable and secure data sharing and exchange can be provided. By further including one or more authorization mechanisms, the data sharing or exchange can be conducted in a more flexible manner with multiple data consuming services from different participants of the battery supply chain accessing the battery data, e.g., battery material configuration data.

It is an object of the present invention to provide an efficient, sustainable and robust way for providing a variable rate application with a high resolution in order to increase the effectivity of the application of treatment products, avoiding unnecessary treating and/or over treatment of the agricultural field, and saving money and amounts of treating products.

These and other objects, which become apparent upon reading the following description, are solved by the subject matters of the independent claims. The dependent claims refer to preferred embodiments of the invention.

The term "battery" may denote any structure configured to store electric power e.g. based on an electrochemical basis. Batteries may be structures comprising a group of two or more cells connected together and are not limited thereto. The battery may include a lithium-ion battery. The at least one component of the battery may refer to any component of the battery, any combination of components of the battery or the battery with all its components. When the term component of the battery it is to be understood the meaning is to be understood that the battery with all its components is included. Component of the battery may be anode active material, cathode active material, electrolyte composition, anode element, cathode element, separator, electrolyte, battery cell, battery module, packaging material, battery management module, cooling module, high-voltage module, wiring, battery housing or the like.

The battery passport may comprise a decentral identifier and a digital representation of battery data. The digital representation may include one or more representation(s) for accessing the battery data or part(s) or subset(s) thereof. The digital representation may include one or more representation(s) of battery data or part(s) or subset(s) thereof. The battery passport may include data related to the battery data, cryptographic information such as a public key and the decentral identifier. The data related to the battery data may include the digital representation of the battery data. The digital representation(s) pointing to battery data or parts thereof may comprise at least one interface to a data providing service. It may further include at least one interface to a data consuming service. It may include an endpoint for data exchange or sharing (resource endpoint) or an endpoint for service interaction (service Endpoint), that is uniquely identified via a communication protocol. The digital representation(s) pointing to battery data or parts thereof may be uniquely associated with the decentral identifier.

The decentral identifier may relate to any identifier uniquely associated with at least one component of the battery. The decentral identifier may include an identifier associated with the battery and/or at least one component of the battery. The decentral identifier may include more than one identifier associated with the battery and/or at least one component of the battery. The decentral identifier may include any unique identifier uniquely associated with a data owner and material configuration data associated with at least one component of the battery to be recycled. The decentral identifier may include a Universally Unique IDentifier (UUID) or a Digital IDentifier (DID). The decentral identifier may be issued by a central or decentral identity issuer. Via the decentral identifier and its unique association with the data owner and material configuration data access to the material configuration data may be controlled by the data owner. The data owner may be any producer of a component of the battery. This contrasts with central authority schemes, where identifiers are provided by the central authority and access to the data is controlled by such central authority. Decentral in this context refers to the usage of the identifier as controlled by the data owner. The decentral identifier may be connected to a digital representation of material configuration data. The digital representation may include a representation for accessing the material configuration data or parts thereof. The decentral identifier may be connected to authentication information and/or authorization information. The decentral identifier may be connected to further battery data. The decentral identifier may comprise any unique identifier uniquely associated with the data owner and battery data. The decentral identifier may include a Universally Unique IDentifier (UUID) or a Digital IDentifier (DID). The decentral identifier may be issued by a central or decentral identity issuer. The decentral identifier may include authentication information. Via the decentral identifier and its unique association with the physical entity and battery data access to the battery data may be controlled by the data owner, .e.g. the producer of the at least one component of the battery. This contrasts with central authority schemes, where identifiers are provided by such central authority and access to data is controlled by such central authority. Decentral in this context refers to the usage of the identifier in implementation as controlled by the data owner.

The term "physical entity" is to be understood broadly in the present case and relates to the physical embodiment of the component of the battery. The physical entity may be any product in the battery supply chain, such as a raw material or basic substance, chemical product or chemical material, component, component assembly or end product. The physical entity may be an electrode active material such as an anode or cathode active material.

The term "data owner" is to be understood broadly in the present case and comprises any entity generating data. Data owner may be the producer of at least one component of the battery. The data generating node may be coupled to the entity owning or producing physical products from or for which data is generated. The data may be generated by a third-party entity on behalf of the entity owning or producing physical products from or for which data is generated. E.g. battery component producer

The term "data consuming service" is to be understood broadly in the present case and comprises computer-executable instructions for accessing and/or processing data, such as battery data, associated with the data owner, e.g., battery component producer or any supply chain participant

The term "data providing service" is to be understood broadly in the present case and comprises computer-executable instructions for providing and/or processing data, such as battery data, associated with the data owner for accessing and/or processing by a data consuming service.

In an embodiment the generation of the battery passport includes providing the decentral identifier associated with a physical entity of at least one component of the battery. In this context the physical entity may relate to a physical product that is associated with the decentral identifier. The physical entity may be any entity in the battery supply chain. The physical entity may relate to at least one material component of the battery, such as electrode active material, cathode active material, anode active material and/or at least one discrete component of the battery, such battery cell, battery pack, battery, or combinations thereof

In an embodiment, the decentral identifier is or is assigned to a physical identifier connected to at least one component of the battery. The connection of the physical identifier with the at least one component of the battery may be provided by means of physical connection to the physical product or physical entity. For instance, the physical identifier may be connected with the physical entity of the at least one component of the battery. The physical entity may be the raw material, such as Cobalt, Nickel or Lithium, intermediate product, such as electrode active material, cathode active material or anode active material or the battery. The physical identifier may have one-to-one correspondence to a virtual identity or to a physical identity by means of a physical connection to the physical entity. In an embodiment , the physical identifier is physically attached to the at least one component of the battery via an identifier element.

The "physical identifier" or "physical identifier element" may refer to any virtual or physical arrangement that associates the decentral identifier with the battery or at least one component of the battery. The physical identifier may be any identifier for the produced component of the battery, such as a batch number or a part number. The physical identifier element may comprise a passive or active element, e.g. QR-code, RFID-tag, but is not limited thereto. The physical identifier element may be a physical identifier physically connected to the battery or at least one component of the battery. The identifier element may include markers embedded in materials, a bar code, a QR-Code, a tag like a RFID tag or similar physical arrangement that allows to digitally identify the battery or at least one component of the battery.

In an embodiment, an identifier element containing the physical identifier is physically attached to the at least one component of the battery. The physical identifier may be provided from a sensor reading a physical identifier element, wherein the physical identifier element is physically connected to the at least one component of the battery. The identification element may be physically connected to the at least one component of the battery, e.g., the battery housing, uniquely identifying the at least one component of the battery. The identification element may be physically connected to any component of the battery, e.g., the packaging of electrode active material, the battery cell or module, uniquely identifying a component of the battery.

In an embodiment, the battery data associated with the decentral identifier includes raw material data. The raw material data may include data signifying virgin or recyclate raw material, raw material properties related to their environmental impact or raw material properties associated with their origin.

In an embodiment, the battery data associated with the decentral identifier includes material configuration data of at least one component of the battery. In an embodiment, the battery data associated with the decentral identifier includes chemical composition data of the electrode active material, e.g., the cathode active material. The chemical composition may relate to one or more constituents and their amounts.

In an embodiment the battery data associated with the decentral identifier includes data related to the properties of the battery material and/or the decentral identifier is associated with data related to the properties of the battery material. Material properties may include physical material properties, e.g. thermodynamic, mechanic, electrodynamic, optic and acoustic material properties, and/or chemical material properties, e.g. standard electrode potential and electronegativity, but are not limited thereto.

The battery data may include the material configuration data specifying material configuration of at least one component of the battery, emission data specifying emissions related to the at least one component of the battery, production data relating to production of the at least one component of the battery, recyclate content data specifying recyclate content of the at least one component of the battery, bio-based content data specifying bio-based content of the at least one component of the battery or combinations or subsets thereof.

Material configuration data may relate to the material configuration of the battery or at least one battery component. The material configuration data may be associated with at least one material type, at least one material property and/or at least one chemical composition associated with at least one component of the battery. Material type, material property and/or chemical composition may specify the material configuration at least in part. Material type may include material specifications like plastics, composite material, metal containing material or the like as present in batteries or components of batteries, such as intermediate products, by-products, or raw material but is not limited thereto. Material properties may include performance, quality, physical material properties, e.g., thermodynamic, mechanic, electrodynamic, optic and acoustic material properties, or chemical material properties, e.g., standard electrode potential and electronegativity, but are not limited thereto. The chemical composition may relate to at least one constituent included in the composition. For instance, the chemical composition may be specified by absolute or relative amount of one or more element(s), chemical compound(s) or constituent(s) that are contained in the material.

The material configuration data may be associated with the material configuration of one or more component(s) of the battery. Material configuration data may specify the material configuration on component basis. For instance, material configuration data may specify at least partially the chemical composition of the electrode active material, the anode active material, the electrolyte or the housing. The material configuration data may be associated with the chemical composition of the electrode active material, e.g., the cathode active material. The battery identifier may be associated with any component including the electrode active material. The component may be the electrode element, such as the anode element or the cathode element, the cell, the cell module or the battery. In one embodiment the material configuration data relates to the chemical composition of the electrode active material. The material configuration data may specify at least one constituent of the chemical composition of the electrode active material, such as transition-metal containing constituents. The at least one decentral identifier may relate to the component of the battery, for which the material configuration data specifies the material configuration. The at least one decentral identifier may relate to a first component of the battery including a second component, for which the material configuration data specifies the material configuration. The at least one decentral identifier may relate to the electrode active material with material configuration, which the material configuration data specifies.

The at least one decentral identifier may relate to the battery, the cell, the cell pack, the electrode element including electrode active material with material configuration, which the material configuration data specifies, the electrode active material or combinations therof. The data related to the electrode active material may include the data related to the raw material as e.g. accessible via the decentral identifier of the raw materials. The data related to the electrode active material may include data related to the material configuration of the electrode active material. The material configuration data may relate to the chemical composition of the electrode active material. The material configuration data may specify at least one constituent of the chemical composition of the electrode active material, such as transition-metal containing constituents. The material configuration data may be associated with the cathode active material. In such an embodiment the material configuration may specify the chemical composition of the cathode active material. Examples are material configurations based on Lithium Nickel Manganese (NCM-type) such as Lithium Nickel Cobalt Manganese Oxide (LiNiCoMnO2), Lithium Iron Phosphate (LPF-type) such as Lithium Iron Phosphate (LiFePO4/C), Lithium Nickel Manganese (LMNO-type) such as Lithium Nickel Manganese Spinel (LiNi0.5Mn1.5O4), Lithium Nickel Cobalt Aluminium (NCA-type) such as Lithium Nickel Cobalt Aluminium Oxide (LiNiCoAlO2), Lithium Manganese (LMO-type) such as Lithium Manganese Oxide (LiMn2O4), Lithium Cobalt (LCO-type) such as Lithium Cobalt Oxide (LiCoO2) or combinations thereof. The material configuration data may be associated with the anode active material. In such an embodiment the material configuration may signify the chemical composition of the anode active material. Examples are natural graphite, artificial graphite, other graphite types or combinations thereof.

Cathode active materials may contain layered oxides (LiMO2 with M=Co, Ni, Mn, Al such as LCO (LiCoO2), NCM (LiNixMnyCozO2), NCA (LiNixCoyAlzO2)), spinels (LiM2O4 with M=Mn, Ni such as LMO (LiMnO4)) or phosphates (LiMPO4 with M= Fe, Mn, Co, Ni such as LiFePO4).

Recyclate content data and/or bio-based content data may comprise any data related to the recyclate content or the bio-based content used for providing or manufacturing the at least one component of the battery or a physical entity at any stage in the battery supply chain. It may be data related to the recyclate content or the bio-based content of an electrode active material such as an anode or cathode active material.

Emission data may comprise any data related to environmental footprint of the at least one component of the battery. The environmental footprint may refer to an entity and its associated environmental footprint. The environmental footprint may be physical entity specific. It may be data specifying emissions related to the at least one component of the battery. It may be data specifying emissions related to electrode active material such as an anode or cathode active material. For instance, the environmental footprint may relate to a product, a company, a process such as a manufacturing process, a raw material or basic substance, a chemical product or material, a component, a component assembly, an end product, combinations thereof or additional entity-specific relations in relation to the at least one component of the battery. Emission data may include data relating to the carbon footprint of a chemical product. Emission data may include data relating to greenhouse gas emissions, e.g., released in production of the battery. Emission data may include data related to greenhouse gas emissions. Greenhouse gas emissions may include emissions such as carbon dioxide (CO2) emission, methane (CH4) emission, nitrous oxide (N2O) emission, hydrofluorocarbons (HFCs) emission, perfluorocarbons (PFCs) emission, sulphurhexafluoride (SF6) emission, nitrogen trifluoride (NF3) emission, combinations thereof and additional emissions. Emission data may include data related to greenhouse gas emissions of an entities or companies own operations (production, power plants and waste incineration). Scope 2 may comprise emissions from energy production which is sourced externally. Scope 3 may comprise all other emissions along the value chain. Specifically, this includes the greenhouse gas emissions of raw materials obtained from suppliers. Product Carbon Footprint (PCF) may sum up greenhouse gas emissions and removals from the consecutive and interlinked process steps related to a particular product. Cradle-to-gate PCF may sum up greenhouse gas emissions based on selected process steps: from the extraction of resources up to the factory gate where the product leaves the company. Such PCFs may be called partial PCFs. In order to achieve such summation, each company providing any products may be able to provide the scope 1 and scope 2 contributions to the PCF for each of its products as accurately as possible, and obtain reliable and consistent data for the PCFs of purchased energy (scope 2) and their raw materials (scope 3).

Production data may comprise any data related to the production of the at least one component of the battery or a product at any stage in the battery supply chain. Preferably production data includes battery production data from the production of at least one component of the battery. Production data may include monitoring and/or control data associated with the production of at least one component of the battery, such as electrode active material, cathode active material, anode active material, a chemical material or chemical product for a component of the battery, a component of the battery, the battery, a component assembly of the battery or a combination thereof. Production data may include measurement data related to a product quality of the at least one component of the battery or a product at any stage in the battery supply chain.

In an embodiment the decentral identifier relates to a material or a physical component of the battery, for which the battery data or the material configuration data specifies the material configuration. In an embodiment the decentral identifier relates to a material or a physical component of the battery, the battery, a cell of the battery, a cell pack of the battery, for which the battery data or the material configuration data specifies the material configuration.

In an embodiment the decentral identifier relates to a first component of the battery including a second component for which the battery data or the material configuration data specifies the material configuration. The decentral identifier may relate to the battery, a cell of the battery, a cell pack of the battery, for which the battery data or the material configuration data specifies the material configuration. The decentral identifier may relate to battery data of a first component, wherein the battery data of the first component comprises battery data of at least a second component of the battery, wherein the battery data of at least the second component specifies the material configuration. The decentral identifier may relate to battery data of the battery, the battery pack and/or the battery cell, wherein the battery data of the battery, the battery pack and/or the battery cell comprises battery data of the battery material, such as electrode active material, wherein the battery data of the battery material, such as electrode active material, specifies the material configuration.

In an embodiment the battery passport for access by a supply chain participant is provided under control of a data providing service associated with a battery component producer. In an embodiment, the decentral identifier is provided by one central node or by one or more decentral nodes. The decentral identifier as generated by one central node or by one or more decentral nodes may be provided to a node generating the battery passport and to at least one authentication data registry node, preferably accessible by the data providing service and/or the data consuming service. This enables customized data sharing or exchange with respect to the battery and the battery supply chain the battery is supplied to. In particular, the data providing service and/or the data consuming service may customize data sharing or exchange protocols based on the anchoring of the decentral identifier to battery data.

In an embodiment, the battery passport includes one or more authentication mechanisms and one or more authorization mechanisms associated with the decentral identifier and the data related to battery data. In particular, the data consuming service, e.g. associated with a first battery component producer, requesting to access the battery data and/or the data provider service, e.g. associated with a second battery component producer such as a downstream supply chain participant, providing access to the chemical process data may be authenticating. Such authentication may be based on the decentral identity and the data related to the authentication mechanism. Based on authorization rules the data provider service, e.g. associated with the second battery component producer such as a downstream supply chain participant, may provide controlled access to part(s) or subset(s) of the battery data to the data consuming service, e.g. associated with the first battery component producer.

In an embodiment of the method for producing the battery component, the assignment of the physical identifier and the decentral identifier is executed through an ID generator running in a production system and/or in a decentral computing system. In an embodiment of the method for producing the battery component, the physical identifier of the battery component is provided by a detector detecting the physical identification element associated with the battery component. In an embodiment of the method for producing the battery component, the battery component is a material component of the battery, preferably an electrode material, more preferably an electrode active material, most preferably cathode active material.

In an embodiment of the method for producing a battery component, the generation of the battery passport includes, gathering or accessing data related to the battery component, preferably electrode active material, more preferably cathode active material, as recorded prior and/or during production of the battery component, preferably electrode active material, more preferably cathode active material. In an embodiment of the method for producing a battery component, the data related to the battery component, preferably electrode active material, more preferably cathode active material includes input or raw material data related to the input or raw material used to produce the battery component, preferably electrode active material, more preferably cathode active material or the data related to the battery component, preferably electrode active material, more preferably cathode active material includes at least one decentral identifier associated with input or raw material data used to produce the battery component, preferably electrode active material, more preferably cathode active material.

### BRIEF DESCRIPTION OF THE DRAWINGS

In the following, the present disclosure is further described with reference to the enclosed figures:
- Fig. 1: illustrates schematically a battery with a battery identification element.
- Fig. 2: illustrates schematically a battery component with an identification element.
- Figs. 3a,b: illustrate example embodiments of a centralized and a decentralized computing environment with computing nodes.
- Fig. 3c: illustrates an example embodiment of a distributed computing environment.
- Fig. 3d: illustrates an example environment for making data of one producer accessible for processing by other producers.
- Fig. 4: illustrates an example of ID-based data for at least one battery component, ID-based battery passport data and an identity manager.
- Fig. 5: illustrates another example of ID-based data for at least one battery component, ID-based battery passport data and an identity manager.
- Fig. 6: illustrates one example of a production facility producing at least one component of the battery associated with a battery passport.
- Fig. 7: illustrates another example of a production facility producing at least one component of the battery associated with a battery passport.
- Figs. 8 to 10: illustrate examples of production systems producing multiple components of the battery associated with one or more battery passport(s).
- Figs. 11 to 13: illustrate examples of battery passports.
- Fig. 14: illustrates one example of using one or more battery passport(s) in a supply chain situation with at least two producers.
- Figs. 15 to 17: illustrate examples of providing access to data via one or more battery passport(s).

### DETAILED DESCRIPTION OF EMBODIMENT

The following embodiments are mere examples for implementing the methods, the systems or the computer elements disclosed herein and shall not be considered limiting.

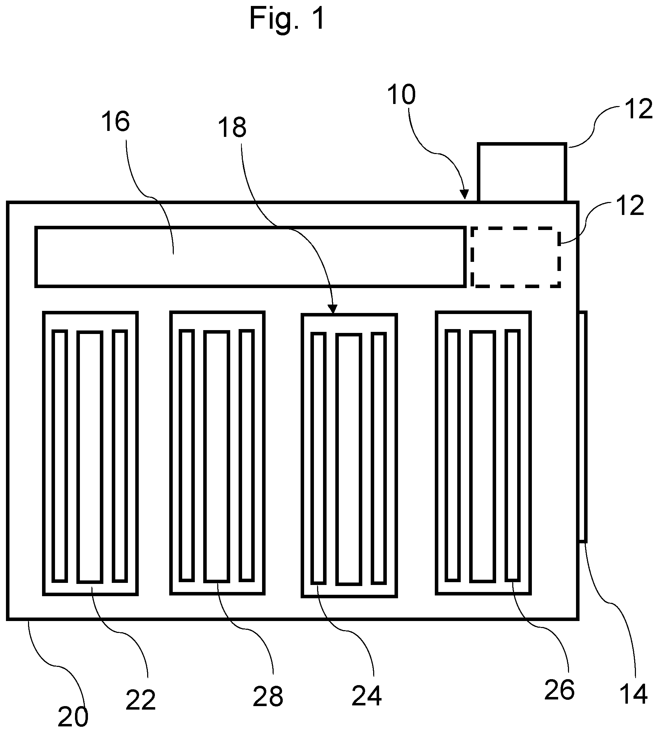

Fig. 1 illustrates schematically a battery 10 with a battery identification element 12, 14.

The battery 10 may comprise a battery management system 16 and a plurality of battery cells 18 arranged inside a battery housing 20. The battery cells 18 may be arranged in battery packs or modules comprising multiple battery cells 18. The battery cell 10 may comprise an electrolyte 22, an anode element 24, a cathode element 26, and a separator 28.

Depending on application, batteries comprise different material compositions for the different components. For instance, the battery may be a lithium-ion battery. The cathode elements 26 may include active material coated on a collector foil such as aluminum or copper foil. Further binders, polyvinylidene fluoride (PVDF) and carbon as conducting agents may be contained. Cathode active materials may contain layered oxides (LiMO2 with M=Co, Ni, Mn, Al such as LCO (LiCoO2), NCM (LiNixMnyCozO2), NCA (LiNixCoyAlzO2)), spinels (LiM2O4 with M=Mn, Ni such as LMO (LiMnO4)) or phosphates (LiMPO4 with M= Fe, Mn, Co, Ni such as LiFePO4).

The anode element 24 may include anode active material coated on collector foil such as aluminum or copper foil. The active material may contain artificial graphite, natural graphite or compositions thereof. Further the active material may include silicon, SiO2, lithium titanate (LTO) or combinations thereof. Further the active material may contain binders such as styrenebutadiene rubber (SBR), polymeric thickener like carboxylmethyl cellulose (CMC) and carbonates as conducting agent.

The electrolytes may comprise salts, solvents and additives such as carbonates, esters or ethers to provide conductivity. Mixtures of cyclic carbonates such as ethylene carbonate (EC) or propylene carbonate (PC) with open chained carbonates such as dimethyl carbonate (DMC) may be included. As conducting salt lithium hexafluorophosphate (LiPF6), lithium bis(trifluoromethyl)sulfonylimid (LiTFSI) and its derivates (e.g., lithium bis(fluorosulfonyl) imide (LiFSI)) or lithium [tris(pentafluorethyl)-trifluorphosphate] (LiFAP), lithium 4,5-dicyano-2-trifluoromethyl-imidazolide (LiTDI), lithium bis(oxalate)borate (LiBOB)), ethyl methyl carbonate (EMC), and/or diethyl carbonate (DEC) may be used.

Separators may divide the space between the electrodes and are permeable for ions. Separator types include: microporous membranes, ceramic-coated separators, non-woven mats, solid inorganic or polymeric electrolytes. Polyolefin-based membranes coated e.g., with PVDF or ceramics may be used.

The battery identification element 12, 14 may be physically associated with the battery 10. The identification element 12, 14 may be physically attached to the battery housing or be part of the battery management system 16. The identification element 12, 14 may be arranged inside or outside the battery housing 20. The identification element 12, 14 may be a passive identification element 14. The passive element 14 may be arranged on the outer surface of the battery housing 20. The passive element 14 may be based on markers embedded into the material. The passive element 14 may include a printed code such as a bar code or a QR code. The identification element 12, 14 may be an active identification element 14. The active element 14 may be a transmitter or transceiver tag, such as an RFID tag enabling communication through e.g. NFC, Bluethoth, Zigbee or other suitable near- to mid-range communication protocols. The identification element 14 may be part of the battery management system 16 or the digital battery identifier may be stored in the battery management system 16.

The battery identification element 12, 14 may be associated with a digital battery identifier. The digital battery identifier may be unique for the battery. The digital battery identifier may be further associated with data relating to the battery identified. Such data may include any data collected during the production or lifetime of the battery 10. For instance, such data may include material data such as material configuration data collected during production of the battery or monitoring data collected during use of the battery may be by associated with the digital battery identifier.

The digital battery identifier may include at least one decentral identifier. The decentral identifier may comprise any unique identifier uniquely associated with the data owner and the identified battery 10. The decentral identifier may include a Universally Unique IDentifier (UUID) or a Digital IDentifier (DID). The decentral identifier may be issued by a central or decentral identity issuer. The decentral identifier may include authentication information for authentication of the data relating to the identified battery. Via the decentral identifier and its unique association with the battery 10 identified access to the data relating to the identified battery may be controlled by at least one data owner. This contrasts with central authority schemes, where identifiers are provided by central authority and access to data is controlled by such central authority. Decentral in this context refers to the usage of the identifier as controlled by any data owner. The data may be hosted in a database associated or under control of the data owner. The identification element 12, 14 may be configured to provide the digital battery identifier for accessing data relating to the identified battery.

In particular, the digital battery identifier may relate to the material configuration data specifying the material configuration of one or more component(s) of the battery. The digital battery identifier may be associated with the battery 10 and the material configuration data may specify the material configuration of one or more component(s) of the battery 10.

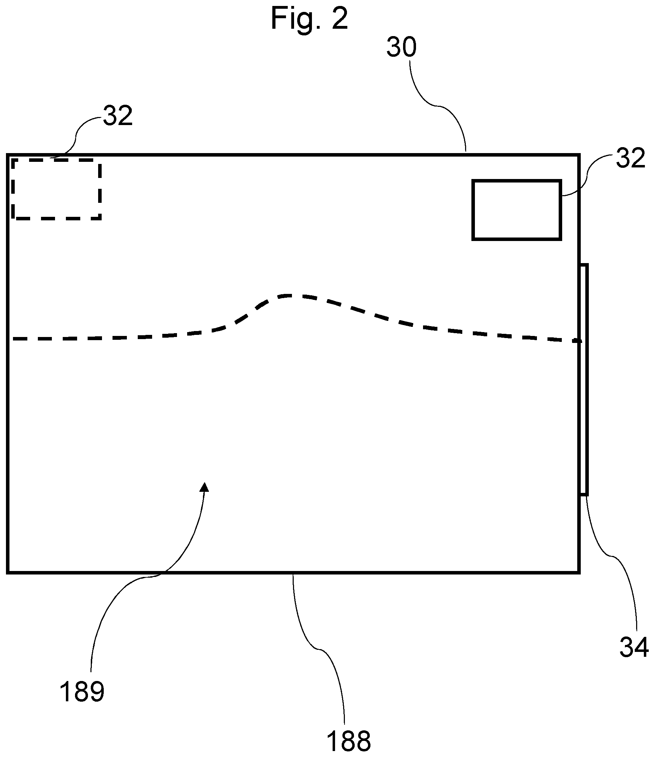

Fig. 2 illustrates schematically a battery component 30 with an identification element 32, 34.

The battery component 30 may include one or more sub-components of the battery 10. The sub-component may include the battery management system 16, the battery housing 20, the battery module or pack with a plurality of battery cells 22, the battery cell 22 or combinations of such sub-components. The battery component identification element 32, 34 may be associated with the battery component 30. The identification element 32, 34 may be physically attached to the battery component 30. The identification element 32, 34 may be arranged inside or outside the battery component 30. The identification element 32, 34 may be a passive or an active identification element 32, 34 as described in the context of Fig. 1. The battery identification element 32, 34 may be associated with a digital battery component identifier. The digital battery identifier may include at least one decentral identifier associated with the identified battery component 30 as described in the context of Fig. 1. In particular, the identification element 32, 34 may be configured to provide the digital battery component identifier for accessing data relating to the identified battery component, such as material configuration data. The battery identifier may relate to the component 30, for which the material configuration data specifies the material configuration.

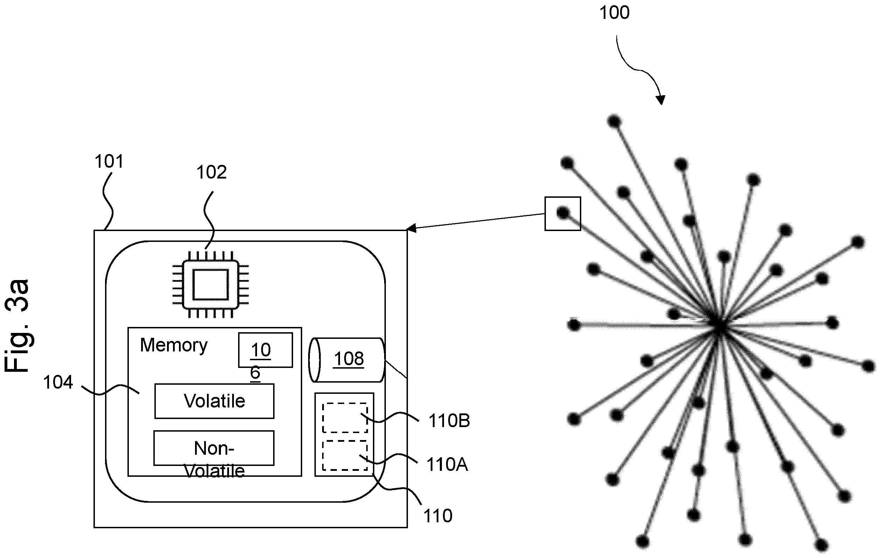

Figs. 3a to 3c illustrate different computing environments, central, decentral and distributed. The methods, apparatuses, systems, uses, computer elements of this disclosure may be implemented in decentral or at least partially decentral computing environments. Providing, determining or processing of data may be realized by different computing nodes, which may be implemented in a centralized, a decentralized or a distributed computing environment.

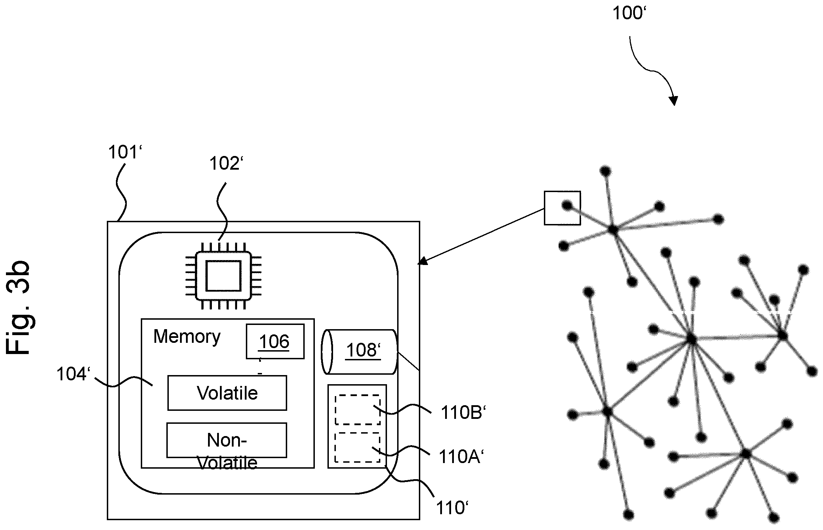

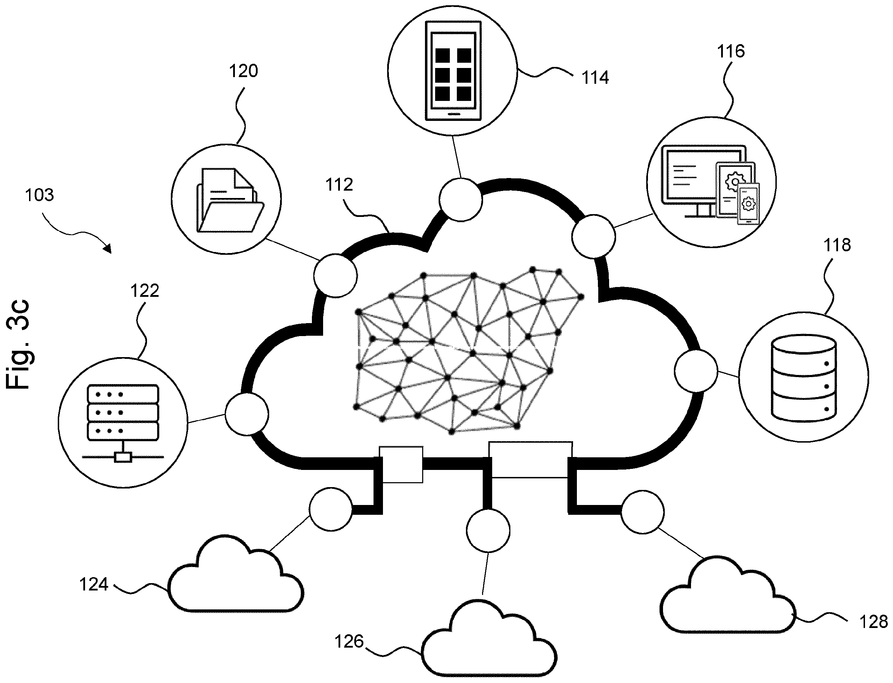

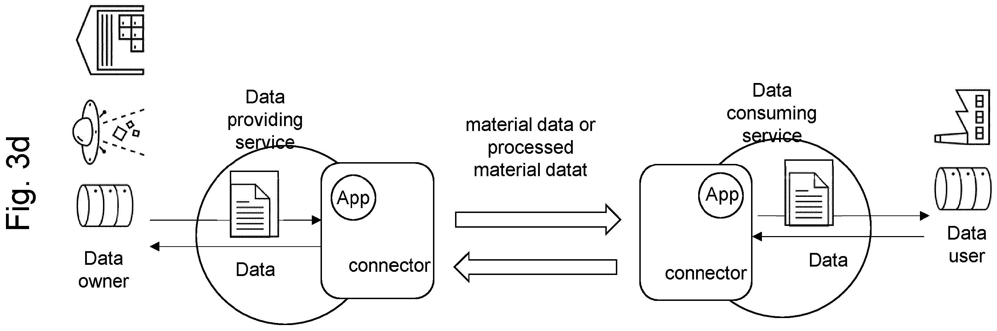

Figs. 3a,b illustrate example embodiments of a centralized and a decentralized computing environment with computing nodes. Fig. 3c illustrates an example embodiment of a distributed computing environment. Fig. 3d illustrates an example environment for making data accessible. Such environment may be implemented according to standard based on International Data Space (IDS) or Digital Identifiers from W3C. Such implementations aid to make data associated with decentral identifiers from material providers accessible to data consumers.

Fig. 3a illustrates an example embodiment of a centralized computing system 100 comprising a central computing node 101 (filled circle in the middle) and several peripheral computing nodes 101.1 to 101.n (denoted as filled circles in the periphery). The term "computing system" is defined herein broadly as including one or more computing nodes, a system of nodes or combinations thereof. The term "computing node" is defined herein broadly and may refer to any device or system that includes at least one physical and tangible processor, and/or a physical and tangible memory capable of having thereon computer-executable instructions that are executed by a processor. Computing nodes are now increasingly taking a wide variety of forms. Computing nodes may, for example, be handheld devices, production facilities, sensors, monitoring systems, control systems, appliances, laptop computers, desktop computers, mainframes, data centers, or even devices that have not conventionally been considered a computing node, such as wearables (e.g., glasses, watches or the like). The memory may take any form and depends on the nature and form of the computing node.

In this example, the peripheral computing nodes 101.1 to 101.n may be connected to one central computing system (or server). In another example, the peripheral computing nodes 101.1 to 101.n may be attached to the central computing node via e.g. a terminal server (not shown). The majority of functions may be carried out by, or obtained from the central computing node (also called remote centralized location). One peripheral computing node 101.n has been expanded to provide an overview of the components present in the peripheral computing node. The central computing node 101 may comprise the same components as described in relation to the peripheral computing node 101.n.

Each computing node 101, 101.1 to 101.n may include at least one hardware processor 102 and memory 104. The term "processor" may refer to an arbitrary logic circuitry configured to perform basic operations of a computer or system, and/or, generally, to a device which is configured for performing calculations or logic operations. In particular, the processor, or computer processor may be configured for processing basic instructions that drive the computer or system. It may be a semi-conductor based processor, a quantum processor, or any other type of processor configures for processing instructions. As an example, the processor may be or may comprise a Central Processing Unit ("CPU"). The processor may be a ("GPU") graphics processing unit, ("TPU") tensor processing unit, ("CISC") Complex Instruction Set Computing microprocessor, Reduced Instruction Set Computing ("RISC") microprocessor, Very Long Instruction Word ("VLIW") microprocessor, or a processor implementing other instruction sets or processors implementing a combination of instruction sets. The processing means may also be one or more special-purpose processing devices such as an Application-Specific Integrated Circuit ("ASIC"), a Field Programmable Gate Array ("FPGA"), a Complex Programmable Logic Device ("CPLD"), a Digital Signal Processor ("DSP"), a network processor, or the like. The methods, systems and devices described herein may be implemented as software in a DSP, in a micro-controller, or in any other side-processor or as hardware circuit within an ASIC, CPLD, or FPGA. It is to be understood that the term processor may also refer to one or more processing devices, such as a distributed system of processing devices located across multiple computer systems (e.g., cloud computing), and is not limited to a single device unless otherwise specified.

The memory 104 may refer to a physical system memory, which may be volatile, non-volatile, or a combination thereof. The memory may include non-volatile mass storage such as physical storage media. The memory may be a computer-readable storage media such as RAM, ROM, EEPROM, CD-ROM, or other optical disk storage, magnetic disk storage, or other magnetic storage devices, non-magnetic disk storage such as solid-state disk or any other physical and tangible storage medium which can be used to store desired program code means in the form of computer-executable instructions or data structures and which can be accessed by the computing system. Moreover, the memory may be a computer-readable media that carries computer- executable instructions (also called transmission media). Further, upon reaching various computing system components, program code means in the form of computer-executable instructions or data structures can be transferred automatically from transmission media to storage media (or vice versa). For example, computer-executable instructions or data structures received over a network or data link can be buffered in RAM within a network interface module (e.g., a "NIC"), and then eventually transferred to computing system RAM and/or to less volatile storage media at a computing system. Thus, it should be understood that storage media can be included in computing components that also (or even primarily) utilize transmission media.

The computing nodes 101, 101.1...101.n may include multiple structures 106 often referred to as an "executable component, executable instructions, computer-executable instructions or instructions". For instance, memory 104 of the computing nodes 101, 101.1...101.n may be illustrated as including executable component 106. The term "executable component" or any equivalent thereof may be the name for a structure that is well understood to one of ordinary skill in the art in the field of computing as being a structure that can be software, hardware, or a combination thereof or which can be implemented in software, hardware, or a combination. For instance, when implemented in software, one of ordinary skill in the art would understand that the structure of an executable component includes software objects, routines, methods, and so forth, that is executed on the computing nodes 101, 101.1...101.n, whether such an executable component exists in the heap of a computing node 101, 101.1...101.n, or whether the executable component exists on computer-readable storage media. In such a case, one of ordinary skill in the art will recognize that the structure of the executable component exists on a computer-readable medium such that, when interpreted by one or more processors of a computing node 101, 101.1...101.n (e.g., by a processor thread), the computing node 101, 101.1...101n is caused to perform a function. Such a structure may be computer-readable directly by the processors (as is the case if the executable component were binary). Alternatively, the structure may be structured to be interpretable and/or compiled (whether in a single stage or in multiple stages) so as to generate such binary that is directly interpretable by the processors. Such an understanding of example structures of an executable component is well within the understanding of one of ordinary skill in the art of computing when using the term "executable component". Examples of executable components implemented in hardware include hardcoded or hard-wired logic gates, that are implemented exclusively or near-exclusively in hardware, such as within a field- programmable gate array (FPGA), an application-specific integrated circuit (ASIC), or any other specialized circuit. In this description, the terms "component", "agent", "manager", "service", "engine", "module", "virtual machine" or the like are used synonymous with the term "executable component.

The processor 102 of each computing node 101, 101.1...101.n may direct the operation of each computing node 101, 101.1...101.n in response to having executed computer-executable instructions that constitute an executable component. For example, such computer-executable instructions may be embodied on one or more computer-readable media that form a computer program product. The computer-executable instructions may be stored in the memory 104 of each computing node 101, 101.1...101.n. Computer-executable instructions comprise, for example, instructions and data which, when executed at a processor 101, cause a general purpose computing node 101, 101.1...101.n, special purpose computing node 101, 101.1...101.n, or special purpose processing device to perform a certain function or group of functions. Alternatively or in addition, the computer-executable instructions may configure the computing node 101, 101.1...101.n to perform a certain function or group of functions. The computer executable instructions may be, for example, binaries or even instructions that undergo some translation (such as compilation) before direct execution by the processors, such as intermediate format instructions such as assembly language, or even source code.

Each computing node 101, 101.1...101.n may contain communication channels 108 that allow each computing node 101.1...101.n to communicate with the central computing node 101, for example, a network (depicted as solid line between peripheral computing nodes and the central computing node in Fig. 25a). A "network" may be defined as one or more data links that enable the transport of electronic data between computing nodes 101, 101.1...101.n and/or modules and/or other electronic devices. When information is transferred or provided over a network or another communications connection (either hardwired, wireless, or a combination of hardwired or wireless) to a computing node 101, 101.1...101.n, the computing node 101, 101.1...101.n properly views the connection as a transmission medium. Transmission media can include a network and/or data links which can be used to carry desired program code means in the form of computer-executable instructions or data structures and which can be accessed by a general-purpose or special-purpose computing nodes 101, 101.1...101.n. Combinations of the above may also be included within the scope of computer-readable media.

The computing node(s) 101, 101.1 to 101.n may further comprise a user interface system 110 for use in interfacing with a user. The user interface system 110 may include output mechanisms 110A as well as input mechanisms 110B. The principles described herein are not limited to the precise output mechanisms 110A or input mechanisms 110B as such will depend on the nature of the device. However, output mechanisms 110A might include, for instance, displays, speakers, displays, tactile output, holograms and so forth. Examples of input mechanisms 110B might include, for instance, microphones, touchscreens, holograms, cameras, keyboards, mouse or other pointer input, sensors of any type, and so forth.

Figure 3b illustrates an example embodiment of a decentralized computing environment 100' with several computing nodes 101.1' to 101.n' denoted as filled circles. In contrast to the centralized computing environment 100 illustrated in Fig. 3a, the computing nodes 101.1' to 101.n' of the decentralized computing environment are not connected to a central computing node 101 and are thus not under control of a central computing node. Instead, resources, both hardware and software, may be allocated to each individual computing node 101.1'...101.n' (local or remote computing system) and data may be distributed among various computing nodes 101.1'...101.n' to perform the tasks. Thus, in a decentral system environment, program modules may be located in both local and remote memory storage devices. One computing node 101' has been expanded to provide an overview of the components present in the computing node 101'. In this example, the computing node 101' comprises the same components as described in relation to Fig. 3a.

Figure 3c illustrates an example embodiment of a distributed computing environment 103. In this description, "distributed computing" may refer to any computing that utilizes multiple computing resources. Such use may be realized through virtualization of physical computing resources. One example of distributed computing is cloud computing. "Cloud computing" may refer a model for enabling on-demand network access to a shared pool of configurable computing resources (e.g., networks, servers, storage, applications, and services). When distributed, cloud computing environments may be distributed internationally within an organization and/or across multiple organizations. In this example, the distributed cloud computing environment 103 may contain the following computing resources: mobile device(s) 114, applications 116, databases 118, data storage 120 and server(s) 122. The cloud computing environment 103 may be deployed as public cloud 124, private cloud 126 or hybrid cloud 128. A private cloud 124 may be owned by an organization and only the members of the organization with proper access can use the private cloud 126, rendering the data in the private cloud at least confidential. In contrast, data stored in a public cloud 126 may be open to anyone over the internet. The hybrid cloud 128 may be a combination of both private and public clouds 124, 126 and may allow to keep some of the data confidential while other data may be publicly available.

Referring back to Figs. 1 and 2, the battery or battery component identifiers provided by the identification element 12, 14, 32, 34 may be processed in an at least partially decentralized computing environment. Battery or battery component identifiers may be configured to access battery or battery component data, such as material data or material configuration data.

Fig. 3d illustrates an example environment for making data accessible according to standards set by the International Data Spaces Association (IDSA; e.g. IDS Reference Architecture Version 3.0 April 2019) or W3C (Decentralized Identifiers (DIDs) v1.0, Core architecture, data model, and representations according to W3C Proposed Recommendation 03 August 2021). Such implementations aid to make data associated with decentral identifiers from material providers accessible to data consumers.

An at least partially decentralized structure based on decentral identifiers allows for novel methods, apparatuses, systems, computer elements, computer executable instructions and their uses in the battery recycling process, which will be described in the following.

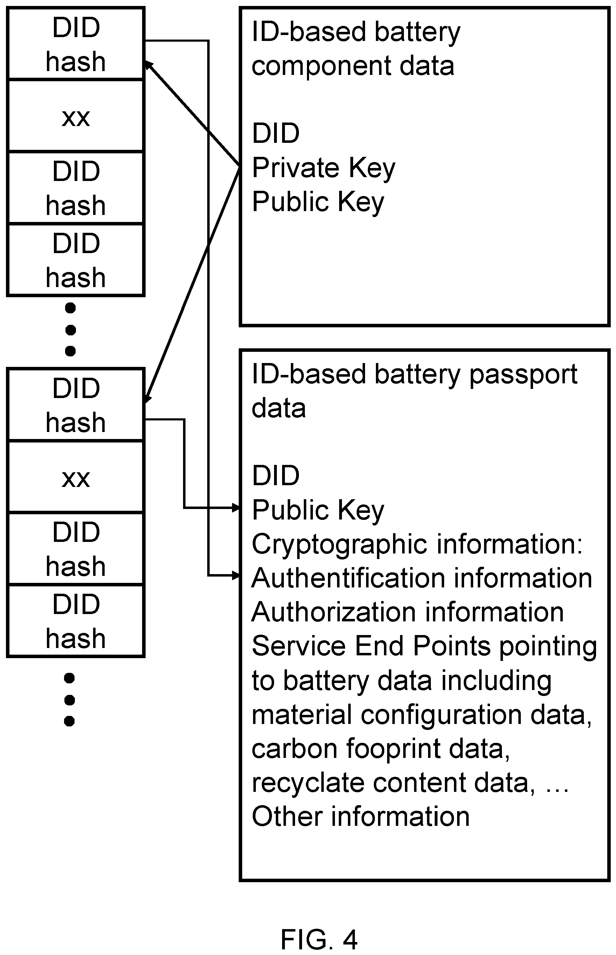

Fig. 4 illustrates an example of ID-based data for at least one battery component, ID-based battery passport data and an identity manager.

The ID may be a decentralized ID (DID). The ID-based battery passport may be a DID document associated with the DID. The ID-based owner data may relate to at least one battery component such as the battery material, the battery cell, the battery cell pack or the battery. The ID-based battery component data may include an ID associated with at least one battery component such as the battery material, the battery cell, the battery cell pack or the battery and may include authentication mechanisms. The ID-based battery component data may include battery data that is electronically associated with the physical entity of at least one battery component. In this context electronically associated may refer to data that is stored in an producer's repository or wallet. Such data may be securely stored and/or managed on a system associated with a production facility, such as an organizational server or client device. The ID-based battery component data may include a DID, a private key and a public key. The ID-based battery component data may be owned and controlled by at least one producer of the at least one battery component. The at least one producer of the at least one battery component may own and control the DID that represents an identity associated with the at least one battery component as DID subject, a private key and public key pair that are associated with the DID. DID may be understood as an identifier and authentication information may be associated with or uniquely linked to such identifier.

The DID subject may be at least one component of the battery including raw material, intermediate product, component, component assembly or end product. The DID subject may be the raw material, the electrode active material, the battery cell, the battery cell pack, the battery or any combination thereof. The DID owner may be a supply chain participant or a manufacturer such as a chemical manufacturer producing chemicals. The DID owner may be an upstream participant in the supply chain of the chemical manufacturer such as a supplier that supplies raw chemical products or precursors to produce chemicals. The DID owner may be a downstream participant in the supply chain of the chemical manufacturer such as a customer that consumes chemicals to produce the intermediate product, the component, the component assembly or the end product. The DID owner may be any participant of the supply chain including raw chemical product supplier, intermediate chemical products manufacturer, intermediate part manufacturer, component manufacturer, component assembly manufacturer, electrode active material manufacturer, battery cell manufacturer, battery cell pack manufacturer, the battery manufacturer or the end product manufacturer.

The DID may be any identifier that is associated with the DID subject and/or the DID owner. Preferably, the identifier is unique to the DID subject and/or DID owner. The identifier may be unique at least within the scope in which the DID is anticipated to be in use. The identifier may be a locally or globally unique identifier for at least one component of the battery including raw material, intermediate product, component, component assembly or end product or a collection thereof; the chemical manufacturer producing chemicals, the upstream participant in the supply chain of the chemical manufacturer, the downstream participant in the supply chain of the chemical manufacturer or a collection thereof; any participant of the supply chain including raw chemical product supplier, intermediate chemical products manufacturer, intermediate part manufacturer, component manufacturer, component assembly manufacturer or end product manufacturer or a collection thereof.

The DID may be a Uniform Resource Identifier (URI) such as a Uniform Resource Locator (URL). The DID may be an Internationalized Resource Identifier (IRI). The DID may be a random string of numbers and letters for increased security. In one embodiment, the DID may be a string of 128 letters and numbers e.g. according to the scheme did:method name: method specific-did such as did:example:ebfeb1f712ebc6f1c276e12ec21. The DID may be decentralized independent of a centralized, third-party management system and under the control of the DID owner.

The battery passport as DID document 16 may be associated with the DID. Accordingly, the battery passport 16 may include a reference to the DID, which is associated with the DID subject that is described by the DID document. The DID document 16 may also an authentication information such as the public key. The public key may be used by third-party entities that are given permission by the DID owner/subject 14 to access information and data owned by the DID owner/subject 14. The public key may also be used for verifying that the DID owner 14, in fact, owns or controls the DID. The DID document 16 may include authentication information, authorization information e.g. to authorize third party entities to read the DID document or some part of the DID document 16 e.g. without giving the third party the right to prove ownership of the DID.

The battery passport may include one or more representations that digitally link to the product data or chemical product data, e.g. by way of service endpoints. A service endpoint may include a network address at which a service operates on behalf of the DID owner. In particular, the service endpoints may refer to services of the DID owner that give access to battery data. Such services may include services to read or analyze battery data. Battery data may include chemical product declaration data, chemical product safety data, certificate of analysis data, emission data, product carbon footprint data, product environmental footprint data, chemical product specification data, product information, technical application data, production data, material configuration data, recyclate content data or combinations thereof.

The battery passport 16 may include various other information such metadata specifying when the battery passport 16 was created, when it was last modified and/or when it expires or when the at least one component of the battery was manufactured or shipped.

The DID and battery passport may be associated with a data registry node such as a centralized data service system or a decentralized data service system, e.g. a distributed ledger or blockchain 10. Possible blockchain systems include Quorum, Hyperledger Fabric. The distributed ledger or blockchain 10 may be used to store a representation of the DID 14 that points to the battery passport 16. A representation of the DID may be stored on distributed computing nodes of the distributed ledger or blockchain 10. For example, DID hash may be stored on multiple computing nodes of the distributed ledger 10 and point to the location of the battery passport 16. In some embodiments, the battery passport 16 may be stored on the distributed ledger. Alternatively, in other embodiments the battery passport 16 may be stored in a data storage (not illustrated) that is associated with the distributed ledger or blockchain 10.

The distributed ledger or blockchain 10 may be any decentralized, distributed network that includes various computing nodes that are in communication with each other. For example, the distributed ledger 10 may include a first distributed computing node 12.1, a second distributed computing node 12.2, a third distributed computing node 12.3, and any number of additional distributed computing node as illustrated by 12.4, 12.5. The distributed ledger or blockchain 10 may operate according to any known standards or methods for distributed ledgers. Examples of conventional distributed ledgers that correspond to the distributed ledger or blockchain 10 include, but are not limited to, Bitcoin [BTC], Ethereum, and Litecoin.

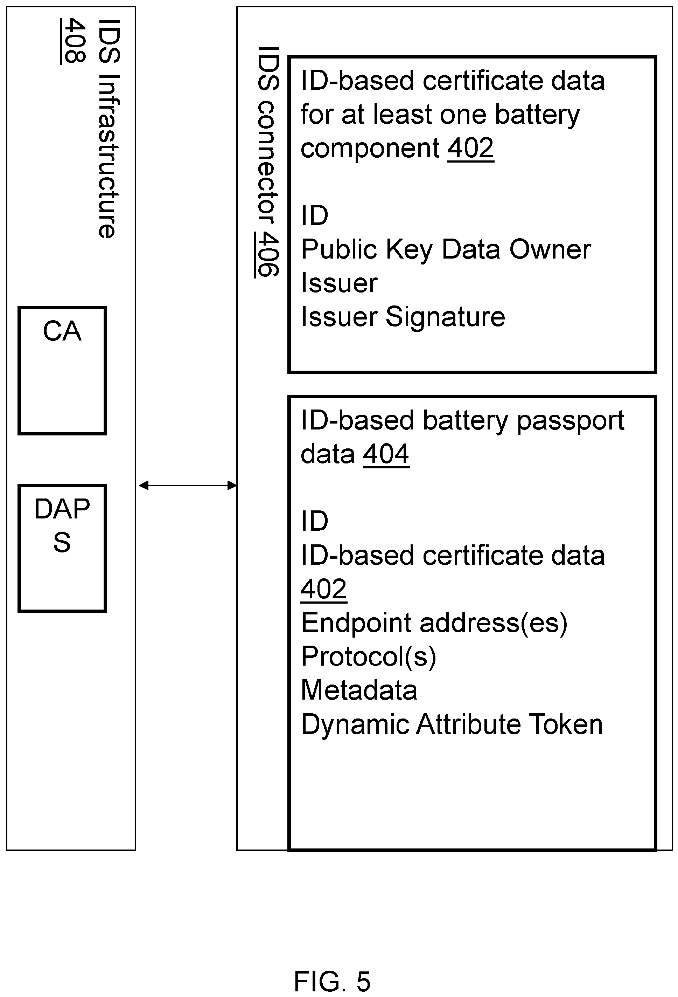

Fig. 5 illustrates another example of ID-based data for at least one battery component, ID-based battery passport data and an identity manager.

The example of Fig. 5 is in essence similar to the example of Fig. 4. The example of Fig. 5 is certificate based with a central identity magaer. ID-based certificate data may include authentication data of the certificate owner and the certificate issuer. For example, a cryptographic signature from the issuer may bind the public key of the data owner to the ID. The identifier may be a locally or globally unique identifier for at least one component of the battery including raw material, intermediate product, component, component assembly or end product or a collection thereof; the chemical manufacturer producing chemicals, the upstream participant in the supply chain of the chemical manufacturer, the downstream participant in the supply chain of the chemical manufacturer or a collection thereof; any participant of the supply chain including raw chemical product supplier, intermediate chemical products manufacturer, intermediate part manufacturer, component manufacturer, component assembly manufacturer or end product manufacturer or a collection thereof. The ID subject may be at least one component of the battery including raw material, intermediate product, component, component assembly or end product. The ID subject may be the raw material, the electrode active material, the battery cell, the battery cell pack, the battery or any combination thereof. The ID owner may be a supply chain participant or a manufacturer such as a chemical manufacturer producing chemicals. The ID owner may be an upstream participant in the supply chain of the chemical manufacturer such as a supplier that supplies raw chemical products or precursors to produce chemicals. The ID owner may be a downstream participant in the supply chain of the chemical manufacturer such as a customer that consumes chemicals to produce the intermediate product, the component, the component assembly or the end product. The ID owner may be any participant of the supply chain including raw chemical product supplier, intermediate chemical products manufacturer, intermediate part manufacturer, component manufacturer, component assembly manufacturer, electrode active material.

The ID may be a unique ID (such as UID) as described in relation to the DID of Figure 2. The certificate may be a X.509 certificate such as X509v3. The ID-based passport data may be associated with the data source of the data owner. The ID-based passport data may include an ID, authentication data and endpoints associated with battery data. Such endpoints may include any digital representation connecting to the data source. The data source may provide or provide access to battery data.

In this certificate-based example, the ID-based battery passport data includes one or more certificate(s) associated with the data owner. The certificates may be associated with an identity manager including e.g. a certificate issuing service and/or a dynamic provisioning service providing dynamic attribute tokens (e.g. OAuth Access Tokens). The information required to verify the certificates are provided via an authentication registry associated with the certificate issuing service and/or a dynamic provisioning service. For instance, in the IDSA Reference Architecture Model, Version 3.0 of April 2019, a connector associated with the data owner, a Certification Authority (CA), a Dynamic Attribute Provisioning Service (DAPS) and a connector associated with the data consumer service are used to verify the identity prior to performing a data exchange (not shown). For this purpose, such connectors include one or more certificate(s) such as X.509 certificate(s). This way the connector possesses a unique identifier embedded in a X.509 certificate that identifies the connector instance.

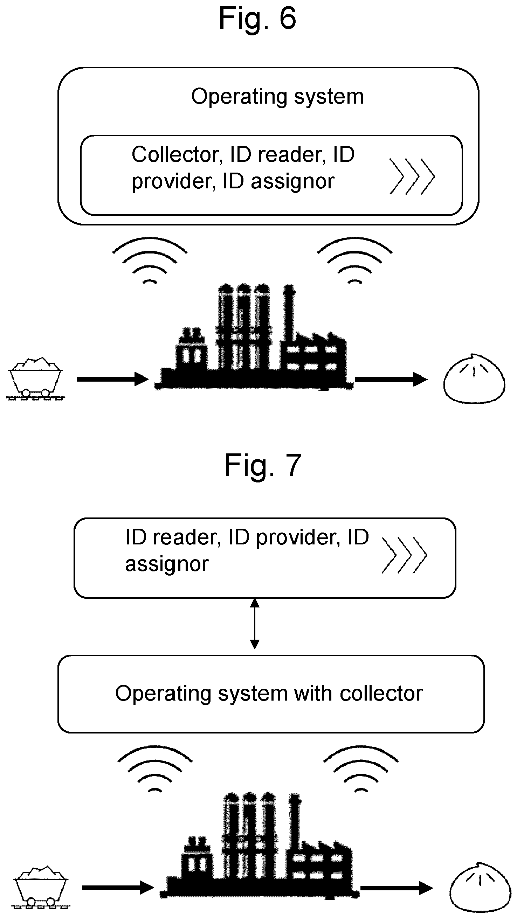

Fig. 6 illustrates one example of a production facility producing at least one component of the battery associated with a battery passport.

The production facility illustrated in Fig. 6 may manufacture at least one component of the battery. The production facility may manufacture electrode active material such as anode or cathode active material. The production facility may include one or more production plants. For example, the production facility for manufacturing of electrode active material, such as cathode active material, may include at least one precursor electrode active material production plant, such as at least one precursor cathode active material (PCAM) plant for processing transition metals to precursors such as transition metal carbonates, oxides or hydroxides, and at least one electrode active material production plant, such as at least one cathode active material (CAM) plant for processing precursors such as transition metal carbonates, oxides or hydroxides to cathode active material. In other embodiments the production facility may manufacture battery cells, battery packs, batteries or end products including the battery.

The physical inputs to the production facility may include materials, such raw materials, intermediate materials or components to be assembled. Raw materials may be virgin or recycled raw materials. In the case of cathode active materials raw materials, such as metals like cobalt, lithium, copper or nickel, may be virgin materials or recycled materials. The physical inputs to the production facility may include discrete battery components, such as battery cells, battery packs or batteries, which may be assembled.

The physical inputs may be associated with the decentral identifier as described in the context of Figs. 4, 5, 11, 12, 13. The physical input(s) may be registered with the production facility. The registration may include providing the decentral identifier associated with the physical inputs. Providing the decentral identifier may include reading the physical identifier element physically connected to the physical input as described in the context of Figs. 1 or 2. Providing the decentral identifier may include accessing a data base with decentral identifiers and fetching the decentral identifier associated with the physical input.

Based on the provided decentral identifier, battery data associated with such decentral identifier may be accessed. Access may be granted through authentication and authorization based on authentication and authorization information associated with the decentral identifier. Based on the decentral identifier, battery data such as chemical product declaration data, chemical product safety data, certificate of analysis data, emission data, product carbon footprint data, product environmental footprint data, chemical product specification data, product information, technical application data, production data, performance data, quality data, material configuration data, recyclate content data or combinations thereof may be accessed. Battery data may be accessed through a data service requesting access to the battery data associated with each decentral identifier and controlled by a physical input data owner. Data owner may be the producer of the physical inputs. The data service may include computer executable instructions operating in an at least partially decentral computing environment. Such computer executable instructions may be based on a Json Web Token (JWT) including authentication information, authorization information and/or a digital representation pointing to battery data or parts thereof. The digital representation may include an endpoint for data exchange or sharing (resource endpoint) or an endpoint for service interaction (service endpoint), that may be uniquely identified via a communication protocol. The digital representation(s) pointing to battery data or parts thereof may be uniquely associated with the decentral identifier. Battery data may be used for the manufacturing process of the production facility.

The production facility may produce the physical output based on one or more physical input(s). The physical output of the production facility may be associated with a physical identifier. The physical output of the production facility may be physically connected to a physical identifier element as described in the context of Figs. 1 and 2. The physical identifier may be assigned to identifier information associated with the decentral identifier. For such assignment the physical identifier element may be read or a data base with physical identifiers may be accessed. A request to provide a decentral identifier may be triggered to assign the physical identifier to the decentral identifier. This way, the decentral identifier may be assigned to the physical output. Providing the decentral identifier may include accessing a data base with decentral identifiers and associated information such as authentication information. Providing the decentral identifier may include accessing a decentral service to provide the decentral identifier and associated information such as authentication information. In response to the request, the battery passport including the decentral identifier and data related to the battery data of the at least one component of the battery may be generated. The data related to the battery data may include a representation such as a pointer or a link to the battery data. The decentral identifier may be associated to data related to the battery data of the physical output. The decentral identifier may further be related or assigned or linked to the decentral identifier of the physical input. The battery passport may be provided to a decentral network for access by other participants or producers of the network. This way the chain of input to output material may be made traceable and usable in further manufacturing steps without exposing the battery data in an uncontrolled manner.

The process steps described above may be executed via an operating system of the production facility. In this embodiment the operating system includes a collector configured to collect battery data and/or physical identifiers as described above. The collector may be configured to collect battery data associated with the component of the battery or battery data associated with the production of the component of the battery, wherein the component of the battery is connected to or comprises the physical identifier. The operating system may in particular include an ID reader configured to provide the physical identity of physical input(s) or output(s) as described above. The system may further include an Assignor configured to assign the decentral identifier and associated information to the physical output as described above. Further the operating system may include an ID provider configured to provide the decentral identifier and associated information as described above.

Fig. 7 illustrates another example of a production facility producing at least one component of the battery associated with a battery passport.

The process steps described in the context of Fig. 6 may be executed via an operating system of the production facility in interaction with the assignor, the collector or reader or the ID provider. In this embodiment the operating system may be communicatively connected to the production facility and the assignor, the collector, the ID reader or the ID provider. The operating system may be configured to provide battery data from the production facility. The operating system may include a collector configured to provide battery data from the production facility. The operating system may include the ID reader configured to read the physical identifier element physically connected to the physical input or output. The Assignor may be configured to assign the decentral identifier and associated information to the physical identifier of the physical output as described above in the context of Fig. 6. The ID provider may be configured to provide the decentral identifier and associated information as described above in the context of Fig. 6. The ID reader may be configured to provide the physical identity and associated information of physical input(s) or output(s) as described above in the context of Fig. 6. The Assignor, the collector, the ID reader and/or the ID provider may be configured as decentral services or applications executed via the decentral network.

Figs. 6 and 7 only show two example embodiments and any combination of the system components shown in Figs. 6 and 7 may be possible. For instance, the ID reader may be configured as part of the operating system, while ID provider and Assignor may not be configured as part of the operating system.

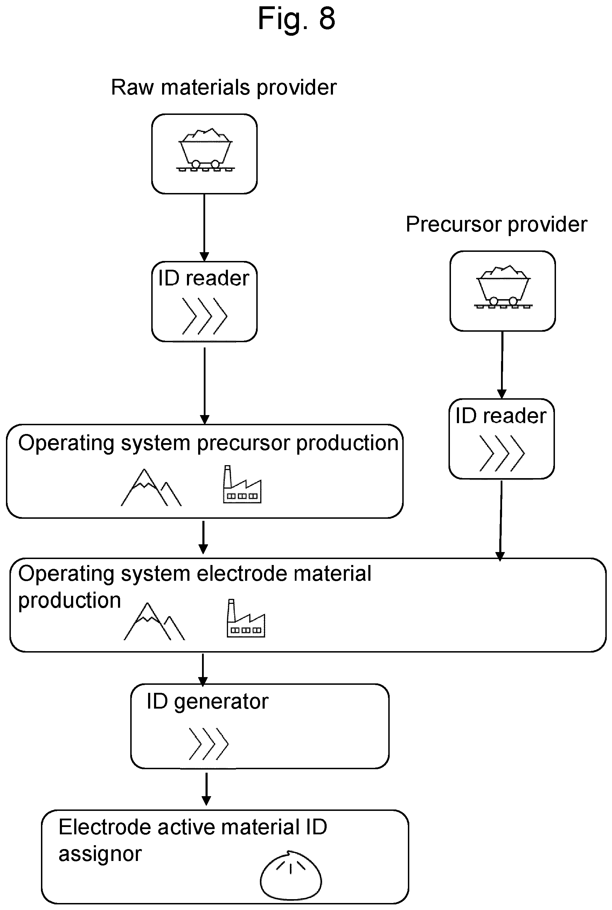

Figs. 8 to 10 illustrate examples for tracking material in the production of a battery from raw materials via electrode active material and the battery to the end product. Figs. 8 specifically illustrates examples for tracking material in the production of electrode active material.

For producing electrode active material raw materials may be provided as physical inputs. The raw materials may comprise transition metals such as cobalt, nickel, manganese. The raw materials may include virgin or recycled materials. The raw materials may be associated with a decentral identifier. The decentral identifier may be associated with a digital twin of the raw materials. The decentral identifier may be associated with raw materials data such as a tag for virgin or recyclate material, material properties related to their environmental impact or material properties associated with their origin.

The production of electrode active material may comprise a two-step process: 1) production of precursor material, 2) production of electrode active material. To produce the precursor material, the raw materials may be used as physical inputs. The operating system of the precursor production may access data related to the raw materials based on the decentral identifier e.g. from a raw materials provider. Such data may be used to operate the production. For instance, if the raw materials are recycled materials, production steps purifying the recyclate may be comprised. For instance, if the raw materials are virgin materials, purification steps may be omitted. The precursor material may be formed by co-precipitating the raw materials such as transition metals as carbonates, oxides or preferably as hydroxides that may or may not be basic.

Production data from precursor production may be stored and/or associated with a decentral identifier.

In a second step the precursor material may be provided to produce electrode active material. The precursor material may comprise the precursor produced by the precursor production. The precursor material may comprise recycled precursor material or precursor material produced by a different entity. Such precursor material may be associated with a decentral identifier via which data related to the precursor material may be accessible.

The precursor may be mixed with a source of lithium such as, but not limited to LiOH, LhO or U2CO3 and calcined (fired) at high temperatures. Lithium salt(s) may be employed as hydrate(s) or in dehydrated form. The calcination - or firing - generally also referred to as thermal treatment or heat treatment of the precursor - may be carried out at temperatures in the range of from 600 to 1000 °C. The thermal treatment may be performed in the heating zone of an oven or kiln. During the thermal treatment a solid-state reaction takes place, and the electrode active material is formed. The electrode active material may be packaged e.g. in powder form.

The produced and packaged electrode active material may be assigned to a decentral identifier and associated information as lined out in Figs. 4, 5, 11, 12, 13. The packaged electrode active material may for instance comprise the physical identifier element, such as a QR-Code, physically attached to the package. Such physical identifier element may be assigned to the decentral identifier. The assignment of physical identifier element and decentral identifier may be executed through an ID generator/assignor running locally, in a decentral system and/or in a distributed system.

For instance, the packaging line may comprise a detector detecting the physical identifier on each package. Based on such recognition the operating system of the electrode active material production may request to provide a decentral identifier and the provided decentral identifier may be assigned to the physical identifier. In response to the request, the battery passport including the decentral identifier and data related to the electrode active material may be generated. For such generation, data related to the electrode active material as recorded prior and/or during production of the electrode active material may be gathered or accessed. Such data may be provided by the operating system of the electrode material production, or a storage environment connect to the operating system of the electrode material production. This may include data collected and stored during precursor material production.

The data related to the electrode active material may include the identifier of raw materials used to produce the electrode active material. The data related to the electrode active material may include the data related to the raw material as e.g. accessible via the decentral identifier of the raw materials. The data related to the electrode active material may include data related to the material configuration of the electrode active material. The material configuration data may relate to the chemical composition of the electrode active material. The material configuration data may specify at least one constituent of the chemical composition of the electrode active material, such as transition-metal containing constituents. The material configuration data may be associated with the cathode active material. In such an embodiment the material configuration may specify the chemical composition of the cathode active material. Examples are material configurations based on Lithium Nickel Manganese (NCM-type) such as Lithium Nickel Cobalt Manganese Oxide (LiNiCoMnO2), Lithium Iron Phosphate (LPF-type) such as Lithium Iron Phosphate (LiFePO4/C), Lithium Nickel Manganese (LMNO-type) such as Lithium Nickel Manganese Spinel (LiNi0.5Mn1.5O4), Lithium Nickel Cobalt Aluminium (NCA-type) such as Lithium Nickel Cobalt Aluminium Oxide (LiNiCoAlO2), Lithium Manganese (LMO-type) such as Lithium Manganese Oxide (LiMn2O4), Lithium Cobalt (LCO-type) such as Lithium Cobalt Oxide (LiCoO2) or combinations thereof.

The data related to the electrode active material may include data related to properties related to environmental impact such as Co2 footprint or recyclate content. For instance, the data related to the electrode active material may specify recyclate content for cobalt, lead, nickel or lithium. Such recyclate content may either be directly associated with the decentral identifier of the electrode active material, or it may be indirectly associated with the decentral identifier of the electrode active material, e.g. via the decentral identifier of the raw materials or the precursor materials. The data related to the electrode active material may include data related to the production conditions as provided by the operating system of the electrode active material production. The data related to the electrode active material may include data related to the operation conditions as provided by the operating system of the electrode active material production. The data related to the electrode active material may include data related to the producer, such as producer name, producer brand or producer identifier. The data related to the electrode active material may include data related to the product, such as product name, product brand or product identifier.

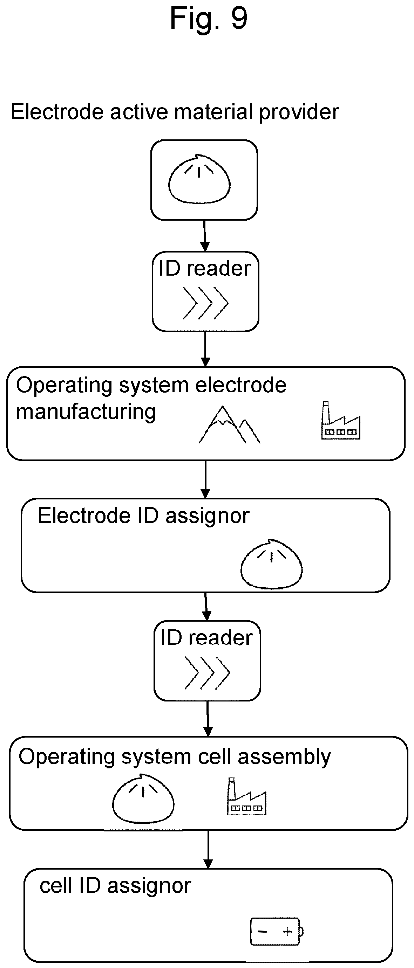

Fig. 9 illustrates an example for tracking material in the production of a battery cell.

For producing battery cells different materials may be provided as physical inputs from material providers. The electrode active material may be associated with a decentral identifier and associated information as lined out in Figs. 4, 5, 11, 12, 13. The decentral identifier may be associated with data related to electrode active material or a digital twin of the electrode active material. The decentralized identifier may be associated with electrode active material data as described in the context of Fig. 8.

The electrode manufacturing may include multiple steps based on input materials. Input materials may be for anode: material such as graphite, conductive carbon black, solvent, binder, and one or more additive(s). Input materials may be for cathode: material such as LMNO, carbon black, solvent and one or more binder(s). The operating system of the electrode production may initiate the production process based on operating parameters for the different process steps. The production process may include an apparatus for dry mixing of active material, additives such as carbon black or binder, an apparatus for wet dispersing by adding solvent, dispersing and homogenizing to a slurry, an apparatus for coating of foil (copper for anode, aluminum for cathode) with slurry on one or both sides, an apparatus for drying, an apparatus for calendaring and/or an apparatus for slitting to produce electrode material, e.g. in the form of roller electrode material.

The operating system of the electrode production may access data related to the electrode active materials or raw materials based on the decentral identifier e.g. from the electrode active materials or raw materials provider. Such data may be used to operate the production.

The produced and packaged electrode may be assigned to a decentral identifier. The packaged electrode may for instance comprise an identifier element, such as a QR Code, physically attached to the package. Such physical identifier element may be assigned to the decentral identifier. The assignment of physical identifier and decentral identifier may be executed through an ID generator running locally, in a decentral system and/or in a distributed system.

For instance, the packaging line may comprise a detector detecting the physical identifier on each package. Based on such recognition the operating system of the electrode production may request to provide a decentral identifier assigned to the physical identifier. In response to the request, the battery passport including the decentral identifier and data related to the electrode active material may be generated. For such generation, data related to the electrode as recorded prior and/or during production of the electrode may be gathered or accessed. Such data may be provided by the operating system of the electrode material production, or a storage environment connect to the operating system of the electrode production.

The data related to the electrode may include the decentral identifier of electrode active materials or raw materials used to produce the electrode active material. The data related to the electrode may include the data related to the electrode active material or raw material as e.g. accessible via the decentral identifier of the electrode active material or raw materials.

For producing batteries or battery cells, the electrode and other components may be provided as physical inputs. The components such as the electrode active material may be associated with a decentral identifier. The decentral identifier may be associated with a digital twin of the components such as the electrode active material. The decentral identifier may be associated with components such as the electrode active material data as described in the context of Fig. 8.

The battery or cell assembly and finishing may include multiple steps based on physical inputs. Input materials may be for anode, electrode, separator, electrolyte, and packaging material. The operating system of the cell production may initiate the production process based on operating parameters for the different process steps. The production process may include an apparatus for separation of electrode and stacking, an apparatus for packaging electrodes and separators, an apparatus for electrolyte filling, an apparatus for degassing and an apparatus for quality control and testing. Data collected during production may be stored.

The operating system of the battery or cell production may access data related to the physical inputs based on the decentral identifier e.g. from an electrode active materials or raw materials provider. Such data may be used to operate the production.

The produced and packaged cell may be assigned to a decentral identifier and associated information as lined out in Figs. 4, 5, 11, 12, 13. The packaged cell or battery may for instance comprise an identifier element, such as a QR Code, physically attached to the package. Such physical identifier element may be assigned to the decentral identifier. The assignment of physical identifier and decentral identifier may be executed through an ID generator running locally, in a decentral system and/or in a distributed system.

For instance, the packaging line may comprise a detector detecting the physical identifier on each package. Based on such recognition the operating system of the cell production may request to provide a decentral identifier assigned to the physical identifier. In response to the request, the battery passport including the decentral identifier and data related to the cell or battery may be generated. For such generation, data related to the cell as recorded prior and/or during production of the cell or battery may be gathered or accessed. Such data may be provided by the operating system of the cell or battery production, or a storage environment connect to the operating system of the electrode production.

The data related to the cell or battery may include the decentral identifier of electrode active materials or raw materials used to produce the electrode active material. The data related to the cell may include the data related to the electrode active material or raw material as e.g. accessible via the decentral identifier of the electrode active material or raw materials.

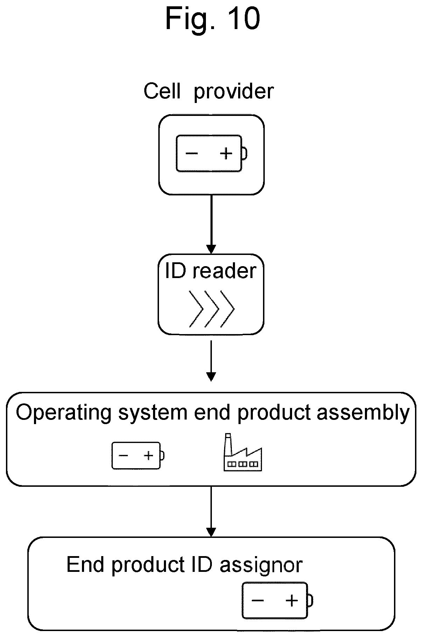

Fig. 10 illustrates an example for tracking material in the production of the end product such as a car or a power tool with the produced battery as lined out in Figs. 8 or 9.

For producing the end product different parts may be provided as inputs. The battery may be associated with the decentral identifier. The decentralized identifier may be associated with a digital twin of the battery and associated information. The decentralized identifier may be associated with cell data as described in the context of Figs. 8 or 9.

The manufacturing may include multiple steps based on discrete input materials. Inputs may be any discrete component.

The operating system of the production may access data related to the battery, the electrode active materials or raw materials based on the decentral identifier e.g. from a electrode active materials or raw materials provider. Such data may be used to operate the production.

The end product may be assigned to a decentral identifier. The end product may for instance comprise an identifier element, such as a QR Code, physically attached to it. Such physical identifier element may be assigned to the decentral identifier. The assignment of physical identifier and decentral identifier may be executed through an ID generator running locally, in a decentral system and/or in a distributed system.

Figs. 9 to 10 are only illustrative examples. Different embodiments for ID generation and their association to digital twins or data related to any component of the battery or the end product are possible.

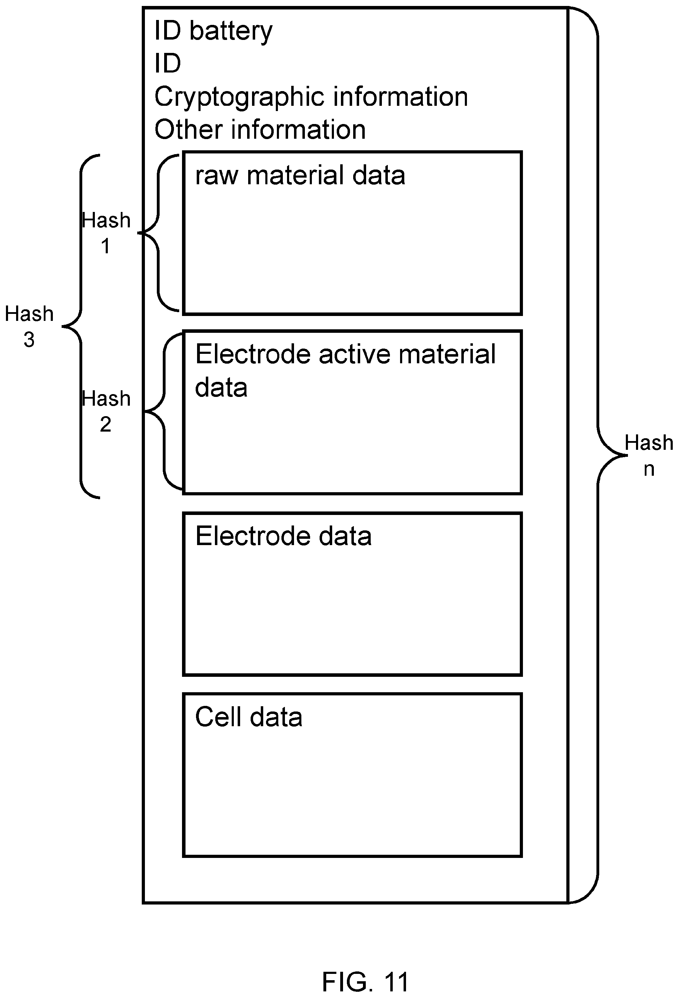

Figs. 11 to 13 illustrate examples of battery passports with different embodiments for connecting decentral identifiers and associated information along the value chain of the battery.

Fig. 11 illustrates one example for tracking material in production of a car based on a single decentral identifier associated with each production output or component for the battery. The decentral identifier may be associated with the raw material, the intermediate material such as electrode active material, the battery cells, the battery and the car with the battery. The ID may be provided by the producer of first or last physical entity. Data related to such physical entities may be related to such single ID and made accessible to other supply chain participants via the decentral network. The connection of the physical entities may be provided via the single ID and the associated data. The ID and the associated data may be hashed individually and/or in connection. For instance, each data set provided in the JWT file may be hashed and the combination of hashes may be hashed. Such hashes may be provided in the decentral network. This way any supply chain participant accessing such data may check data corruption by hashing the received data associated with the ID and matching the hashes.

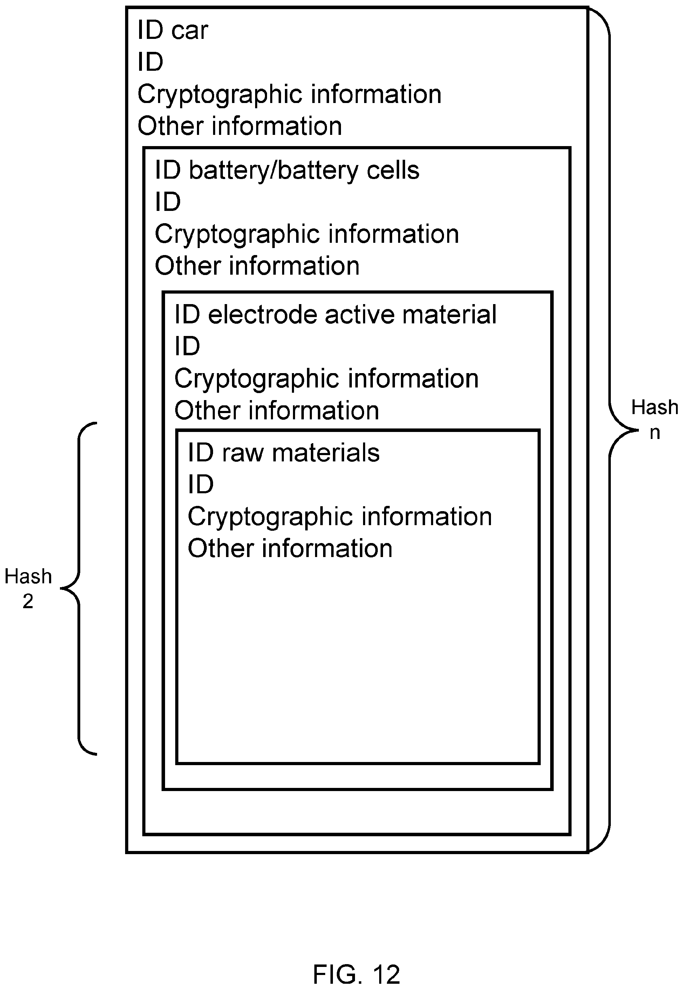

Fig. 12 illustrates another example for tracking material in production of a car based on multiple decentral identifiers associated with the car identifier. The set of IDs may be generated in each production step along the value chain and IDs may be linked in a hierarchical order lining out the embedding. The connection of the physical entities may be provided via the hierarchy of IDs and the associated data. The IDs and the associated data may be hashed individually and/or in connection. For instance, each data set provided in the JWT file may be hashed and the combination of hashes may be hashed. Such hashes may be provided in the decentral network. This way any supply chain participant accessing such data may check data corruption by hashing the received data associated with the IDs and matching the hashes.

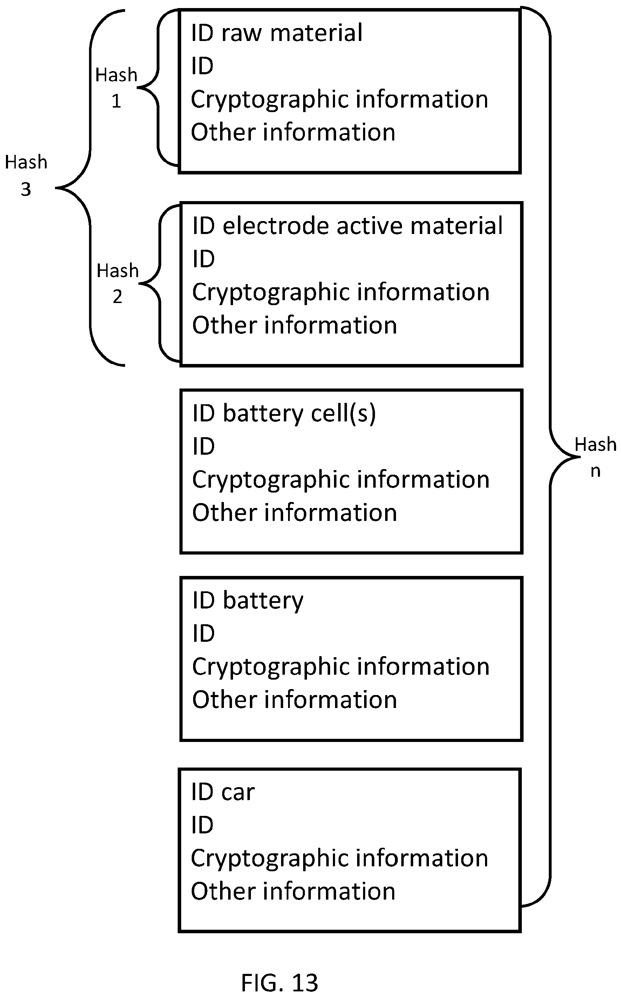

Fig. 13 illustrates another example for tracking material in production of a car based on multiple decentral identifiers associated with the car identifier. The set of IDs may be generated in each production step along the value chain and IDs may be linked, but fully separate. Data related to physical entities may be related to individual IDs per physical entity and made accessible to other supply chain participants via the decentral network. The connection of the physical entities may be provided via the individual IDs and the associated data. The individual IDs and the associated data may be hashed individually and/or in connection. The connection of hashed may provide the connection of physical entities in the supply chain. For instance, each data set provided in the JWT file may be hashed and the combination of hashes may be hashed. Such hashes may be provided in the decentral network. This way any supply chain participant accessing such data may check data corruption by hashing the received data associated with the ID and matching the hashes. Further the connected hashed may provide a way to track the physical entities through the supply chain.

The embodiments illustrated in Figs. 11 to 13 are only examples and multiple combinations of direct and indirect ID connection via hashing, linking or other mechanism are possible.

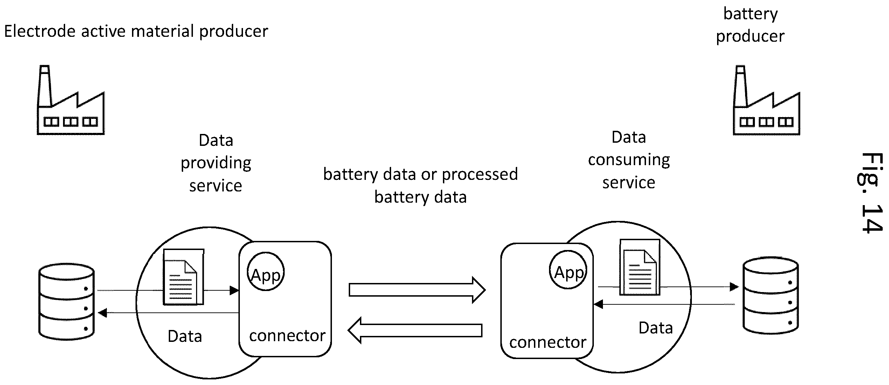

Fig. 14 illustrates one example of using one or more battery passport(s) in a supply chain situation with at least two producers.

In the example of Fig. 14 the producer may be the cathode active material producer. This is only an example and the embodiment lined out here may be equally valid for any other material producer in the battery supply chain.

The cathode active material producer may store environmental impact data, such as carbon footprint data. The cathode active material producer may store recyclate data from production such as recyclate content of cobalt, nickel, lithium, lead or the like. The cathode active material producer may store data related to production such as production date, site, batch number, producer identity, or the like. The cathode active material producer may store data related to the material configuration or the chemical composition.

The cathode active material producer may provide access to the data related to the electrode active material, that the cathode active material producer provided to a battery producer. Such data sharing may be conducted by way of the decentral network, where the decentral data provider may provide the data and the data consumer may consume the data directly or indirectly.

For accessing in data related to the electrode active material, the service component on the battery producer side may use the public piece of the cryptographic information such as the public key of the electrode active material producer and the battery producer. Based on cryptographic information and an authentication protocol the access by the service component on the battery producer side may be authenticated. In a further step the service component on the battery producer side may request access to the data related to the electrode active material or a subset of such data. The data or the subset may be linked to authorization rules. Based on such authorization rules the access by the service component on the battery producer side may be granted or denied. If access is granted data may be transferred or processed from the service component on the battery producer side as provided by the service component on the electrode active material producer side.

This way a secure and controlled sharing of data may be realized. In particular, the data remains under control by the data owner, while battery data may be shared with selected participants of the network. The battery data including material configuration may be made available to production participants along the value chain. This allows for property tracking and aggregation, appropriate handling in production, testing or quality control.

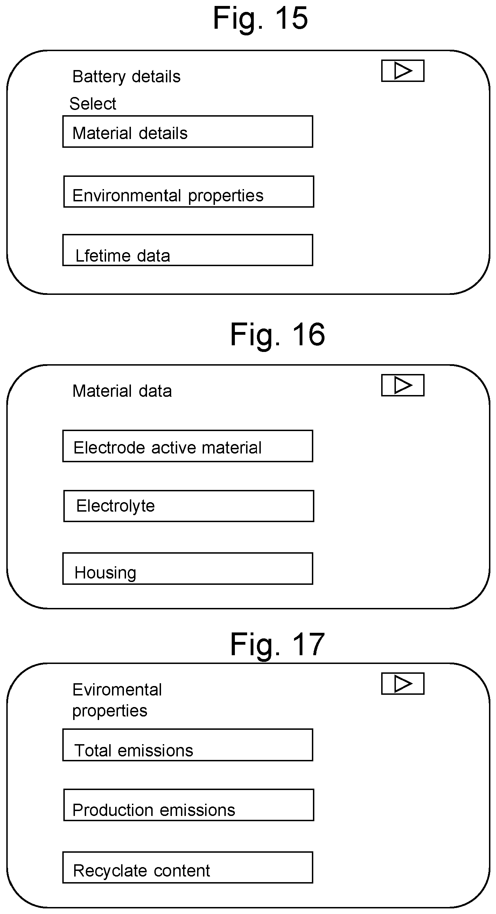

Figs. 15 to 17 illustrate examples of providing access to data via one or more battery passport(s).

Via the decentral setup described above different supply chain participants or producers up to end consumers may access data from supply chain participants or producers. Access to such data may include transferring the data to the requester or processing the data and transferring the processed result to the requester. The example of Fig. 15 shows data that may be accessed based on the decentral identifier of the car or the battery. Material data may be provided from different material providers, that have supplied material for the production of the battery or the car. Environmental impact data may be provided aggregated from different material providers, that have participated in the production of the battery or the car. Lifetime data may be provided as collected during use of the end product.

The example of Fig. 16 shows material data such as electrode active material data as provided by the electrode active material producer, electrolyte data as provided by the electrolyte producer, housing data as provided by the housing producer.

The example of Fig. 17 shows environmental data such as total emissions data as aggregated from data provided by producers of components of the car or battery, production emission data as provided by producers of components of the car or battery, recyclate content data as provided by producers of components of the car or battery.

## Claims

1. An apparatus for generating a battery passport, the apparatus comprising:
one or more computing nodes and one or more computer-readable media having thereon computer-executable instructions that are structured such that, when executed by the one or more computing nodes, cause the apparatus to perform the following steps:
collecting battery data associated with a battery or battery data associated with a production of the battery, wherein the battery is connected to or comprises a physical identifier receiving a request to provide at least one decentral identifier associated with the battery data of the battery;
in response to the request, generating the battery passport including the at least one decentral identifier and data related to the battery data of the battery, wherein the data related to the battery data includes a digital representation of the battery data, wherein the digital representation includes one or more representation(s) for accessing the battery data or part(s) or subset(s) thereof, wherein the generation of the battery passport includes providing the at least one decentral identifier associated with a physical entity of the battery and uniquely associated with the battery and the battery data, wherein the physical identifier is assigned to the at least one decentral identifier for generating the battery passport associated with the battery,
providing the battery passport to a decentral network for access by other participants or producers of the decentral network,
wherein via the at least one decentral identifier and its unique association with the battery and the battery data access to the battery data is controlled by a data owner, wherein the battery data is hosted in a database under control of the data owner.

2. The apparatus according to claim 1, wherein the at least one decentral identifier is or is assigned to the physical identifier connected to the battery, wherein an identifier element containing the physical identifier is physically attached to the battery.

3. The apparatus according to any one of the preceding claims, wherein the battery data associated with the at least one decentral identifier includes raw material data.

4. The apparatus according to any one of the preceding claims, wherein the battery data associated with the at least one decentral identifier includes material configuration data of at least one component of the battery, preferably wherein the battery data associated with the at least one decentral identifier includes chemical composition data of an electrode active material.

5. The apparatus according to any one of the preceding claims, wherein the battery data associated with the at least one decentral identifier includes data related to properties of battery materia and/or the at least one decentral identifier is associated with data related to properties of battery material.

6. The apparatus according to any one of the preceding claims, wherein the battery data includes material configuration data specifying material configuration of at least one component of the battery, emission data specifying emissions related to the at least one component of the battery, production data relating to production of the at least one component of the battery, recyclate content data specifying recyclate content of the at least one component of the battery, bio-based content data specifying bio-based content of the at least one component of the battery or combinations or subsets thereof.

7. The apparatus according to any one of the preceding claims, wherein the at least one decentral identifier relates to a material or a physical component of the battery, for which the battery data or a material configuration data specifies a material configuration.

8. The apparatus according to any one of the preceding claims, wherein the at least one decentral identifier relates to battery data of a first component of the battery, wherein the battery data of the first component of the battery comprises battery data of at least a second component of the battery, wherein the battery data of at least the second component specifies a material configuration.

9. The apparatus according to any one of the preceding claims, wherein the battery passport includes one or more authentication mechanisms and one or more authorization mechanisms associated with the at least one decentral identifier and the data related to battery data.

10. A computer-implemented method for generating a battery passport, the method comprising:
collecting battery data associated with a battery or battery data associated with a production of the battery, wherein the battery is connected to or comprises a physical identifier receiving a request to provide at least one decentral identifier associated with the battery data of the battery;
in response to the request, generating the battery passport including the at least one decentral identifier and data related to the battery data of the battery, wherein the data related to the battery data includes a digital representation of the battery data, wherein the digital representation includes one or more representation(s) for accessing the battery data or part(s) or subset(s) thereof, wherein the generation of the battery passport includes providing the at least one decentral identifier associated with a physical entity of the battery and uniquely associated with the battery and the battery data, wherein the physical identifier is assigned to the at least one decentral identifier for generating the battery passport associated with the battery,
providing the battery passport to a decentral network for access by other participants or producers of the decentral network
wherein via the at least one decentral identifier and its unique association with the battery and battery data access to the battery data is controlled by a data owner, wherein the battery data is hosted in a database under control of the data owner.

11. A system for producing or providing a battery associated with a battery passport, the system comprising:
a production line configured to produce the battery connected to or comprising a physical identifier,
an apparatus for generating a battery passport according to any one of claims 1 to 9.

12. A method for producing or providing a battery associated with a battery passport, the method comprising:
producing the battery connected to or comprising a physical identifier by a production line configured to produce the battery connected to or comprising a physical identifier,
generating the battery passport via an operating system of the production line, wherein the battery passport is generated according to the method of claim 10.

13. A battery associated with a battery passport, wherein the battery passport includes one or more decentral identifier(s) associated with battery data of the battery and data related to the battery data, wherein the data related to the battery data includes a digital representation of the battery data, wherein the digital representation includes one or more representation(s) for accessing the battery data or part(s) or subset(s) thereof, wherein the one or more decentral identifier(s) are assigned to a physical identifier associated with the battery, and wherein the battery is produced and the battery passport is generated according to the method of claim 12 or by the system according to claim 11.

14. A computer program element with instructions, which when executed on one or more computing node(s) cause the one or more computing node(s) to carry out the steps of the method of claim 10.

15. A use of a battery passport as generated according to the method of claim 10 or 12 or by the apparatus according to any one of claims 1 to 9 or the system according to claim 11 to further process a battery associated with the battery passport, wherein the battery passport includes one or more decentral identifier(s) associated with battery data of the battery and data related to the battery data, wherein the data related to the battery data includes a digital representation of the battery data, wherein the digital representation includes one or more representation(s) for accessing the battery data or part(s) or subset(s) thereof, wherein the one or more decentral identifier(s) are assigned to a physical identifier associated with the battery.

## Patentansprüche

1. Vorrichtung zum Erzeugen eines Batteriepasses, wobei die Vorrichtung Folgendes umfasst:
einen oder mehrere Rechenknoten und ein oder mehrere computerlesbare Medien mit darauf befindlichen computerausführbaren Anweisungen, die so strukturiert sind, dass sie, wenn sie durch den einen oder die mehreren Rechenknoten ausgeführt werden, die Vorrichtung veranlassen, die folgenden Schritte durchzuführen:
Sammeln von Batteriedaten, die mit einer Batterie assoziiert sind, oder von Batteriedaten, die mit einer Herstellung der Batterie assoziiert sind, wobei die Batterie mit einer physischen Kennung verbunden ist oder eine physische Kennung umfasst, Empfangen einer Anforderung zum Bereitstellen mindestens einer dezentralen Kennung, die mit den Batteriedaten der Batterie assoziiert ist;
als Reaktion auf die Anforderung, Erzeugen des Batteriepasses einschließlich der mindestens einen dezentralen Kennung und Daten, die sich auf die Batteriedaten der Batterie beziehen, wobei die Daten, die sich auf die Batteriedaten beziehen, eine digitale Repräsentation der Batteriedaten beinhalten, wobei die digitale Repräsentation eine oder mehrere Repräsentation(en) zum Zugreifen auf die Batteriedaten oder einen Teil (Teile) oder eine Teilmenge (Teilmengen) davon beinhaltet, wobei die Erzeugung des Batteriepasses Bereitstellen der mindestens einen dezentralen Kennung beinhaltet, die mit einer physischen Entität der Batterie assoziiert ist und eindeutig mit der Batterie und den Batteriedaten assoziiert ist, wobei die physische Kennung der mindestens einen dezentralen Kennung zum Erzeugen des Batteriepasses, der mit der Batterie assoziiert ist, zugewiesen ist,
Bereitstellen des Batteriepasses für ein dezentrales Netz zum Zugriff durch andere Teilnehmer oder Hersteller auf das dezentrale Netz;
wobei über die mindestens eine dezentrale Kennung und ihre eindeutige Zuordnung zu der Batterie und den Batteriedaten der Zugriff auf die Batteriedaten durch einen Dateneigentümer gesteuert wird, wobei die Batteriedaten in einer Datenbank unter Steuerung des Dateneigentümers gehostet werden.

2. Vorrichtung nach Anspruch 1, wobei die mindestens eine dezentrale Kennung die mit der Batterie verbundene physische Kennung ist oder dieser zugewiesen ist, wobei ein Kennungselement, das die physische Kennung enthält, physisch an der Batterie angebracht ist.

3. Vorrichtung nach einem der vorhergehenden Ansprüche, wobei die Batteriedaten, die mit der mindestens einen dezentralen Kennung assoziiert sind, Rohstoffdaten beinhalten.

4. Vorrichtung nach einem der vorhergehenden Ansprüche, wobei die Batteriedaten, die mit der mindestens einen dezentralen Kennung assoziiert sind, Materialkonfigurationsdaten mindestens eines Bestandteils der Batterie beinhalten, vorzugsweise wobei die Batteriedaten, die mit der mindestens einen dezentralen Kennung assoziiert sind, chemische Zusammensetzungsdaten eines elektrodenaktiven Materials beinhalten.

5. Vorrichtung nach einem der vorhergehenden Ansprüche, wobei die Batteriedaten, die mit der mindestens einen dezentralen Kennung assoziiert sind, Daten beinhalten, die sich auf Eigenschaften von Batteriematerial beziehen, und/oder die mindestens eine dezentrale Kennung mit Daten assoziiert ist, die sich auf Eigenschaften von Batteriematerial beziehen.

6. Vorrichtung nach einem der vorhergehenden Ansprüche, wobei die Batteriedaten Materialkonfigurationsdaten, die eine Materialkonfiguration mindestens eines Bestandteils der Batterie spezifizieren, Emissionsdaten, die Emissionen spezifizieren, die sich auf den mindestens einen Bestandteil der Batterie beziehen, Herstellungsdaten, die sich auf die Herstellung des mindestens einen Bestandteils der Batterie beziehen, Recyclatinhaltsdaten, die einen Recyclatinhalt des mindestens einen Bestandteils der Batterie spezifizieren, biobasierte Inhaltsdaten, die biobasierten Inhalt des mindestens einen Bestandteils der Batterie spezifizieren, oder Kombinationen oder Teilmengen davon beinhalten.

7. Vorrichtung nach einem der vorhergehenden Ansprüche, wobei sich die mindestens eine dezentrale Kennung auf ein Material oder einen physischen Bestandteil der Batterie bezieht, für das oder den die Batteriedaten oder Materialkonfigurationsdaten eine Materialkonfiguration spezifizieren.

8. Vorrichtung nach einem der vorhergehenden Ansprüche, wobei sich die mindestens eine dezentrale Kennung auf Batteriedaten eines ersten Bestandteils der Batterie bezieht, wobei die Batteriedaten des ersten Bestandteils der Batterie Batteriedaten mindestens eines zweiten Bestandteils der Batterie umfassen, wobei die Batteriedaten mindestens des zweiten Bestandteils eine Materialkonfiguration spezifizieren.

9. Vorrichtung nach einem der vorhergehenden Ansprüche, wobei der Batteriepass einen oder mehrere Authentifizierungsmechanismen und einen oder mehrere Autorisierungsmechanismen beinhaltet, die mit der mindestens einen dezentralen Kennung und den Daten, die sich auf die Batteriedaten beziehen, assoziiert sind.

10. Computerimplementiertes Verfahren zum Erzeugen eines Batteriepasses, wobei das Verfahren Folgendes umfasst:
Sammeln von Batteriedaten, die mit einer Batterie assoziiert sind, oder von Batteriedaten, die mit einer Herstellung der Batterie assoziiert sind, wobei die Batterie mit einer physischen Kennung verbunden ist oder eine physische Kennung umfasst, Empfangen einer Anforderung zum Bereitstellen mindestens einer dezentralen Kennung, die mit den Batteriedaten der Batterie assoziiert ist;
als Reaktion auf die Anforderung, Erzeugen des Batteriepasses einschließlich der mindestens einen dezentralen Kennung und Daten, die sich auf die Batteriedaten der Batterie beziehen, wobei die Daten, die sich auf die Batteriedaten beziehen, eine digitale Repräsentation der Batteriedaten beinhalten, wobei die digitale Repräsentation eine oder mehrere Repräsentation(en) zum Zugreifen auf die Batteriedaten oder einen Teil (Teile) oder eine Teilmenge (Teilmengen) davon beinhaltet, wobei die Erzeugung des Batteriepasses Bereitstellen der mindestens einen dezentralen Kennung beinhaltet, die mit einer physischen Entität der Batterie assoziiert ist und eindeutig mit der Batterie und den Batteriedaten assoziiert ist, wobei die physische Kennung der mindestens einen dezentralen Kennung zum Erzeugen des Batteriepasses, der mit der Batterie assoziiert ist, zugewiesen ist,
Bereitstellen des Batteriepasses für ein dezentrales Netz zum Zugriff durch andere Teilnehmer oder Hersteller auf das dezentrale Netz,
wobei über die mindestens eine dezentrale Kennung und ihre eindeutige Assoziation mit der Batterie und Batteriedaten der Zugriff auf die Batteriedaten durch einen Dateneigentümer gesteuert wird, wobei die Batteriedaten in einer Datenbank unter Steuerung des Dateneigentümers gehostet werden.

11. System zum Herstellen oder Bereitstellen einer Batterie, die mit einem Batteriepass assoziiert ist, wobei das System Folgendes umfasst:
eine Fertigungsstraße, die dazu ausgelegt ist, die Batterie herzustellen, die mit einer physischen Kennung verbunden ist oder diese umfasst,
eine Vorrichtung zum Erzeugen eines Batteriepasses nach einem der Ansprüche 1 bis 9.

12. Verfahren zum Herstellen oder Bereitstellen einer Batterie, die mit einem Batteriepass assoziiert ist, wobei die Vorschrift Folgendes umfasst:
Herstellen der Batterie, die mit einer physischen Kennung verbunden ist oder diese umfasst, durch eine Fertigungsstraße, die dazu ausgelegt ist, die Batterie herzustellen, die mit einer physischen Kennung verbunden ist oder diese umfasst,
Erzeugen des Batteriepasses über ein Betriebssystem der Fertigungsstraße, wobei der Batteriepass gemäß dem Verfahren nach Anspruch 10 erzeugt wird.

13. Batterie, die mit einem Batteriepass assoziiert ist, wobei der Batteriepass eine oder mehrere dezentrale Kennung(en) beinhaltet, die mit Batteriedaten der Batterie und Daten assoziiert sind, die sich auf die Batteriedaten beziehen, wobei die Daten, die sich auf die Batteriedaten beziehen, eine digitale Repräsentation der Batteriedaten beinhalten, wobei die digitale Repräsentation eine oder mehrere Repräsentation(en) zum Zugreifen auf die Batteriedaten oder einen Teil (Teile) oder eine Teilmenge (Teilmengen) davon beinhaltet, wobei der eine oder die mehreren dezentralen Kennung(en) einer physischen Kennung zugeordnet sind, die mit der Batterie assoziiert ist, und wobei die Batterie hergestellt wird und der Batteriepass erzeugt wird gemäß dem Verfahren nach Anspruch 12 oder durch das System nach Anspruch 11.

14. Computerprogrammelement mit Anweisungen, die, wenn sie auf einem oder mehreren Rechenknoten durchgeführt werden, bewirken, dass der eine oder die mehreren Rechenknoten die Schritte des Verfahrens nach Anspruch 10 ausführen.

15. Anwendung eines Batteriepasses, wie gemäß dem Verfahren nach Anspruch 10 oder 12 oder durch die Vorrichtung nach einem der Ansprüche 1 bis 9 oder das System nach Anspruch 11 erzeugt, um eine Batterie, die mit dem Batteriepass assoziiert ist, weiter zu verarbeiten, wobei der Batteriepass eine oder mehrere dezentrale Kennung(en) beinhaltet, die mit Batteriedaten der Batterie und Daten assoziiert sind, die sich auf die Batteriedaten beziehen, wobei die Daten, die sich auf die Batteriedaten beziehen, eine digitale Repräsentation der Batteriedaten beinhalten, wobei die digitale Repräsentation eine oder mehrere Repräsentation(en) zum Zugreifen auf die Batteriedaten oder einen Teil (Teile) oder eine Teilmenge (Teilmengen) davon beinhaltet, wobei die eine oder die mehreren dezentralen Kennung(en) einer physischen Kennung zugewiesen sind, die mit der Batterie assoziiert ist.

## Revendications

1. Appareil destiné à générer un passeport de batterie, l'appareil comprenant :
un ou plusieurs nœuds informatiques et un ou plusieurs supports lisibles par ordinateur portant des instructions exécutables par ordinateur qui sont structurées de telle sorte que, lors de leur exécution par le ou les nœuds informatiques, elles amènent l'appareil à réaliser les étapes suivantes :
collecte de données de batterie associées à une batterie ou de données de batterie associées à une production de la batterie, la batterie étant connectée à ou comprenant un identifiant physique recevant une requête de fourniture d'au moins un identifiant décentralisé associé aux données de batterie de la batterie ;
en réponse à la requête, génération du passeport de batterie comportant l'au moins un identifiant décentralisé et des données relatives aux données de batterie de la batterie, les données relatives aux données de batterie comportant une représentation numérique des données de batterie, la représentation numérique comportant une ou plusieurs représentations permettant d'accéder aux données de batterie ou à une ou des parties ou sous-ensembles de celles-ci, la génération du passeport de batterie comportant la fourniture de l'au moins un identifiant décentralisé associé à une entité physique de la batterie et associé de façon unique à la batterie et aux données de batterie, l'identifiant physique étant assigné à l'au moins un identifiant décentralisé pour générer le passeport de batterie associé à la batterie,
fourniture du passeport de batterie à un réseau décentralisé pour que d'autres participants ou producteurs du réseau décentralisé y accèdent,
dans lequel, par le biais de l'au moins un identifiant décentralisé et de son association unique avec la batterie et les données de batterie, l'accès aux données de batterie est contrôlé par un propriétaire de données, les données de batterie étant hébergées dans une base de données sous le contrôle du propriétaire de données.

2. Appareil selon la revendication 1, dans lequel l'au moins un identifiant décentralisé est ou est assigné à l'identifiant physique connecté à la batterie, un élément d'identification contenant l'identifiant physique étant attaché physiquement à la batterie.

3. Appareil selon l'une quelconque des revendications précédentes, dans lequel les données de batterie associées à l'au moins un identifiant décentralisé comportent des données sur des matières premières.

4. Appareil selon l'une quelconque des revendications précédentes, dans lequel les données de batterie associées à l'au moins un identifiant décentralisé comportent des données de configuration de matériau d'au moins un composant de la batterie, de préférence dans lequel les données de batterie associées à l'au moins un identifiant décentralisé comportent des données sur la composition chimique d'un matériau actif d'électrode.

5. Appareil selon l'une quelconque des revendications précédentes, dans lequel les données de batterie associées à l'au moins un identifiant décentralisé comportent des données relatives aux propriétés des matériaux de la batterie et/ou l'au moins un identifiant décentralisé est associé à des données relatives aux propriétés des matériaux de la batterie.

6. Appareil selon l'une quelconque des revendications précédentes, dans lequel les données de batterie comportent des données de configuration de matériau spécifiant la configuration du matériau d'au moins un composant de la batterie, des données d'émission spécifiant les émissions relatives à l'au moins un composant de la batterie, des données de production relatives à la production de l'au moins un composant de la batterie, des données sur le contenu recyclé spécifiant le contenu recyclé de l'au moins un composant de la batterie, des données sur le contenu biosourcé spécifiant le contenu biosourcé de l'au moins un composant de la batterie, ou des combinaisons ou sous-ensembles de celles-ci.

7. Appareil selon l'une quelconque des revendications précédentes, dans lequel l'au moins un identifiant décentralisé se rapporte à un matériau ou un composant physique de la batterie pour lequel les données de batterie ou des données de configuration de matériau spécifient une configuration de matériau.

8. Appareil selon l'une quelconque des revendications précédentes, dans lequel l'au moins un identifiant décentralisé se rapporte à des données de batterie sur un premier composant de la batterie, les données de batterie sur le premier composant de la batterie comprenant des données de batterie sur au moins un deuxième composant de la batterie, les données de batterie sur l'au moins un deuxième composant spécifiant une configuration de matériau.

9. Appareil selon l'une quelconque des revendications précédentes, dans lequel le passeport de batterie comporte un ou plusieurs mécanismes d'authentification et un ou plusieurs mécanismes d'autorisation associés à l'au moins un identifiant décentralisé et aux données relatives aux données de batterie.

10. Procédé mis en œuvre par ordinateur pour générer un passeport de batterie, le procédé comprenant :
la collecte de données de batterie associées à une batterie ou de données de batterie associées à une production de la batterie, la batterie étant connectée à ou comprenant un identifiant physique recevant une requête de fourniture d'au moins un identifiant décentralisé associé aux données de batterie de la batterie ;
en réponse à la requête, la génération du passeport de batterie comportant l'au moins un identifiant décentralisé et des données relatives aux données de batterie de la batterie, les données relatives aux données de batterie comportant une représentation numérique des données de batterie, la représentation numérique comportant une ou plusieurs représentations permettant d'accéder aux données de batterie ou à une ou des parties ou sous-ensembles de celles-ci, la génération du passeport de batterie comportant la fourniture de l'au moins un identifiant décentralisé associé à une entité physique de la batterie et associé de façon unique à la batterie et aux données de batterie, l'identifiant physique étant assigné à l'au moins un identifiant décentralisé pour générer le passeport de batterie associé à la batterie,
la fourniture du passeport de batterie à un réseau décentralisé pour que d'autres participants ou producteurs du réseau décentralisé y accèdent,
dans lequel, par le biais de l'au moins un identifiant décentralisé et de son association unique avec la batterie et les données de batterie, l'accès aux données de batterie est contrôlé par un propriétaire de données, les données de batterie étant hébergées dans une base de données sous le contrôle du propriétaire de données.

11. Système destiné à produire ou fournir une batterie associée à un passeport de batterie, le système comprenant :
une ligne de production conçue pour produire la batterie connectée à ou comprenant un identifiant physique,
un appareil destiné à générer un passeport de batterie selon l'une quelconque des revendications 1 à 9.

12. Procédé destiné à produire ou fournir une batterie associée à un passeport de batterie, le procédé comprenant :
la production de la batterie connectée à ou comprenant un identifiant physique par une ligne de production conçue pour produire la batterie connectée à ou comprenant un identifiant physique,
la génération du passeport de batterie par un système d'exploitation de la ligne de production, le passeport de batterie étant généré selon le procédé de la revendication 10.

13. Batterie associée à un passeport de batterie, le passeport de batterie comportant un ou plusieurs identifiants décentralisés associés à des données de batterie de la batterie et des données relatives aux données de batterie, les données relatives aux données de batterie comportant une représentation numérique des données de batterie, la représentation numérique comportant une ou plusieurs représentations permettant d'accéder aux données de batterie ou à une ou des parties ou sous-ensembles de celles-ci, le ou les identifiants décentralisés étant assignés à un identifiant physique associé à la batterie, et la batterie étant produite et le passeport de batterie étant généré selon le procédé de la revendication 12 ou par le système selon la revendication 11.

14. Élément de programme informatique avec des instructions qui, lors de leur exécution sur un ou plusieurs nœuds informatiques, amènent le ou les nœuds informatiques à réaliser les étapes du procédé de la revendication 10.

15. Utilisation d'un passeport de batterie tel que généré selon le procédé de la revendication 10 ou 12 ou par l'appareil selon l'une quelconque des revendications 1 à 9 ou le système selon la revendication 11 pour réaliser un traitement supplémentaire sur une batterie associée au passeport de batterie, le passeport de batterie comportant un ou plusieurs identifiants décentralisés associés à des données de batterie de la batterie et des données relatives aux données de batterie, les données relatives aux données de batterie comportant une représentation numérique des données de batterie, la représentation numérique comportant une ou plusieurs représentations permettant d'accéder aux données de batterie ou à une ou des parties ou sous-ensembles de celles-ci, le ou les identifiants décentralisés étant assignés à un identifiant physique associé à la batterie.
